# EUROPEAN PATENT APPLICATION

(11) **EP 4 403 634 A1**
(43) Date of publication of application: **24.07.2024**
(21) Application number: 22870304.7
(22) Date of filing: 15.09.2022
(51) Int. Cl.: C12N 15/113, A61K 31/7105, A61K 48/00, A61P 35/00

(54) **ANTISENSE COMPOUND THAT REGULATES EXPRESSION OF WFDC2**

(30) Priority: 16.09.2021 KR 20210124349
(71) Applicant: Qmine Co., Ltd., Seoul 03759 (KR)
(72) Inventor: KIM, Yeon Joon, Seoul 05502 (KR); KIM, Ji Eun, Suwon-si Gyeonggi-do 16708 (KR)
(74) Representative: Cabinet Nony
(86) International application number: PCT/KR2022/013783
(87) International publication number: WO 2023/043220

(57) **Abstract**

The present invention relates to antisense compounds that modulate expression of WFDC2. The antisense compounds that modulate expression of WFDC2 according to the present invention may exhibit an anticancer effect on various cancer types.

## Description

### Technical Field

The present invention relates to antisense compounds that modulate expression of WFDC2.

### Background Art

WFDC2 is a glycosylated protein that was first observed in human epididymal tissue, and has been reported to be overexpressed in various cancers, including ovarian cancer. The WFDC2 gene product is a member of the family of stable 4-disulfide core proteins. In studies on the WFDC2 protein and the gene encoding the same, human epididymis-specific cDNA encodes a protein with sequence homology to extracellular protease inhibitors, and comparative hybridization of an array of ovarian cDNAs has been performed for the discovery of genes overexpressed in ovarian carcinoma. Also, molecular characterization of epididymal proteins has been performed, and cloning and analysis of mRNA specifically expressed in human epididymis have been performed. Through these studies, overexpression of WFDC2 suggests that the protein can be used as a biomarker for cancer, especially ovarian cancer.

U.S. Patent No. 7,811,778 relates to a method for diagnosing gastrointestinal cancer, and discloses that one of up-regulated genes whose expression is increased significantly during transdifferentiation of chief cells into SPEM after oxyntic atrophy is WFDC2.

In addition, Korean Patent No. 10-2055305 relates to a marker for diagnosis and targeted treatment of gastroesophageal border adenocarcinoma, and discloses that WFDC2, one of various genes whose expression level increases, is a gene whose expression measures the Bayesian Compound Covariate Predictor (BCCP) score, and has the potential to be a biomarker for diagnosing gastric cancer or esophageal cancer.

As such, there are prior art documents showing that WFDC2 is one of various genes whose expression increases during carcinogenesis and can be used as a biomarker for ovarian cancer, gastric cancer, etc. However, few studies have been conducted to confirm the cancer therapeutic effect of antisense compounds that inhibit or suppress expression of WFDC2.

### DISCLOSURE

### Technical Problem

One aspect of the present invention provides an antisense compound comprising a modified oligonucleotide that is complementary to a nucleotide sequence in a transcript of a gene encoding WFDC2 (WAP Four-Disulfide Core Domain 2) and consists of 10 to 30 linked nucleosides.

Another aspect of the present invention provides a conjugate in which the antisense compound is covalently linked to at least one non-nucleotide moiety.

Still another aspect of the present invention provides a pharmaceutical composition for preventing or treating cancer comprising the antisense compound or the conjugate as an active ingredient.

### Technical Solution

One aspect of the present invention is intended to provide an antisense compound comprising a modified oligonucleotide that is complementary to a nucleotide sequence in a transcript of a gene encoding WFDC2 (WAP Four-Disulfide Core Domain 2) and consists of 10 to 30 linked nucleosides.

According to one embodiment, the nucleotide sequence of the transcript of the gene encoding WFDC2 may be SEQ ID NO: 1 or SEQ ID NO: 2.

According to one embodiment, the antisense compound may comprise a modified oligonucleotide consisting of 16 to 20 linked nucleosides.

According to one embodiment, the modified oligonucleotide may comprise at least one modification selected from among at least one modified internucleoside linkage, at least one modified nucleoside comprising a modified sugar moiety, and at least one modified nucleoside comprising a modified nucleobase.

According to one embodiment, the modified nucleoside may comprise at least one modified sugar moiety selected from the group consisting of sugar moieties substituted with 2'-O-methyl, 2'-O-methoxyethyl, 2'-amino, 2'-fluoro, 2'-arabino-fluoro, 2'-O-benzyl, or 2'-O-methyl-4-pyridine.

According to one embodiment, the modified nucleoside may be at least one modified nucleoside selected from the group consisting of locked nucleic acid (LNA), constrained ethyl bicyclic nucleic acid (cEt), 2'-O,4'-C-ethylene-bridged nucleic acid (ENA), and tricyclo-DNA.

According to one embodiment, the modified nucleoside may be a modified nucleoside comprising a sugar surrogate having a six-membered ring or an acyclic moiety.

According to one embodiment, the modified nucleoside may be a modified nucleoside comprising at least one modified nucleobase selected from the group consisting of pseudouridine, 2'-thiouridine, N6'-methyladenosine, 5'-methylcytidine, 5'-fluoro-2-deoxyuridine, N-ethylpiperidine 7'-EAA triazol modified adenine, N-ethylpiperidine 6'-triazol modified adenine, 6'-phenylpyrrolocytosine, 2',4'-difluorotoluylribonuleoside, and 5'-nitroindole.

According to one embodiment, the modified internucleoside linkage may be at least one modified internucleoside linkage selected from the group consisting of phosphotriester, phosphoramidate, mesyl phosphoramidate, phosphorothioate, phosphorodithioate, methylphosphonate, and methoxypropyl-phosphonate.

According to one embodiment, the modified oligonucleotide may comprise a gap segment consisting of linked deoxynucleosides, a 5' wing segment consisting of linked nucleosides, and a 3' wing segment consisting of linked nucleosides, wherein the gap segment may be positioned between the 5' wing segment and the 3' wing segment and wherein the nucleoside of each wing segment may comprise a modified sugar moiety or a sugar surrogate.

According to one embodiment, the modified oligonucleotide may comprise a gap segment consisting of 8 to 10 linked deoxynucleosides;
a 5' wing segment consisting of 3 to 5 linked nucleosides; and
a 3' wing segment consisting of 3 to 5 linked nucleosides, wherein the gap segment may be positioned between the 5' wing segment and the 3' wing segment and wherein the nucleoside of each wing segment may comprise a modified sugar moiety.

According to one embodiment, the antisense compound may have a nucleotide sequence that is at least 70%, at least 80%, at least 90% or fully complementary to any sequence of SEQ ID NO: 1 or SEQ ID NO: 2, and the antisense compound may comprise a modified oligonucleotide having a nucleotide sequence comprising at least 8 contiguous nucleobases fully complementary to any portion of an oligonucleotide sequence selected from the group consisting of start site 25 to stop site 46, start site 284 to stop site 305, start site 520 to stop site 545, start site 2222 to stop site 2344, start site 7334 to stop site 9301, start site 9506 to stop site 9551, start site 9733 to stop site 10143, start site 10271 to stop site 10302, start site 10360 to stop site 10905, start site 10977 to stop site 11292, start site 11448 to stop site 11563, and start site 11633 to stop site 11773 of the nucleotide sequence of SEQ ID NO: 1, wherein the modified oligonucleotide is able to reduce any one or more of the mRNA level and protein level of WFDC.

According to one embodiment, the antisense compound may comprise a modified oligonucleotide complementary to SEQ ID NO: 1 or SEQ ID NO: 2, wherein the antisense compound comprises the modified oligonucleotide with a nucleotide sequence comprising at least 8 contiguous nucleobases that perfectly match any one oligonucleotide sequence selected from the group consisting of SEQ ID NO: 7, SEQ ID NO: 8, SEQ ID NO: 9, SEQ ID NO: 20, SEQ ID NO: 38, SEQ ID NO: 39, SEQ ID NO: 42, SEQ ID NO: 43, SEQ ID NO: 46, SEQ ID NO: 47, SEQ ID NO: 48, SEQ ID NO: 49, SEQ ID NO: 65, SEQ ID NO: 83, SEQ ID NO: 86, SEQ ID NO: 89, SEQ ID NO: 109, SEQ ID NO: 113, SEQ ID NO: 119, SEQ ID NO: 120, SEQ ID NO: 121, SEQ ID NO: 122, SEQ ID NO: 123, SEQ ID NO: 129, SEQ ID NO: 131, SEQ ID NO: 135, SEQ ID NO: 136, SEQ ID NO: 140, SEQ ID NO: 141, SEQ ID NO: 142, SEQ ID NO: 148, SEQ ID NO: 154, SEQ ID NO: 155, SEQ ID NO: 156, SEQ ID NO: 157, SEQ ID NO: 165, SEQ ID NO: 169, SEQ ID NO: 176, SEQ ID NO: 191, SEQ ID NO: 205, SEQ ID NO: 210, SEQ ID NO: 213, SEQ ID NO: 214, SEQ ID NO: 215, SEQ ID NO: 218, SEQ ID NO: 228, SEQ ID NO: 229, SEQ ID NO: 237, SEQ ID NO: 238, SEQ ID NO: 239, SEQ ID NO: 240, SEQ ID NO: 243, SEQ ID NO: 244, SEQ ID NO: 245, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 264, SEQ ID NO: 282, SEQ ID NO: 284, SEQ ID NO: 285, SEQ ID NO: 286, SEQ ID NO: 287, SEQ ID NO: 289, SEQ ID NO: 290, SEQ ID NO: 291, SEQ ID NO: 291, SEQ ID NO: 292, SEQ ID NO: 293, SEQ ID NO: 295, SEQ ID NO: 300, SEQ ID NO: 310, SEQ ID NO: 313, SEQ ID NO: 314, SEQ ID NO: 316, SEQ ID NO: 317, SEQ ID NO: 320, SEQ ID NO: 330, SEQ ID NO: 331, SEQ ID NO: 336, SEQ ID NO: 343, SEQ ID NO: 376, SEQ ID NO: 377, SEQ ID NO: 378, SEQ ID NO: 379, SEQ ID NO: 380, SEQ ID NO: 381, SEQ ID NO: 382, and SEQ ID NO: 383, wherein the modified oligonucleotide is able to reduce any one or more of the mRNA level and protein level of WFDC.

According to one embodiment, the antisense compound may be a modified oligonucleotide having any one nucleotide sequence selected from the group consisting of SEQ ID NO: 7, SEQ ID NO: 8, SEQ ID NO: 9, SEQ ID NO: 20, SEQ ID NO: 38, SEQ ID NO: 39, SEQ ID NO: 42, SEQ ID NO: 43, SEQ ID NO: 46, SEQ ID NO: 47, SEQ ID NO: 48, SEQ ID NO: 49, SEQ ID NO: 65, SEQ ID NO: 83, SEQ ID NO: 86, SEQ ID NO: 89, SEQ ID NO: 109, SEQ ID NO: 113, SEQ ID NO: 119, SEQ ID NO: 120, SEQ ID NO: 121, SEQ ID NO: 122, SEQ ID NO: 123, SEQ ID NO: 129, SEQ ID NO: 131, SEQ ID NO: 135, SEQ ID NO: 136, SEQ ID NO: 140, SEQ ID NO: 141, SEQ ID NO: 142, SEQ ID NO: 148, SEQ ID NO: 154, SEQ ID NO: 155, SEQ ID NO: 156, SEQ ID NO: 157, SEQ ID NO: 165, SEQ ID NO: 169, SEQ ID NO: 176, SEQ ID NO: 191, SEQ ID NO: 205, SEQ ID NO: 210, SEQ ID NO: 213, SEQ ID NO: 214, SEQ ID NO: 215, SEQ ID NO: 218, SEQ ID NO: 228, SEQ ID NO: 229, SEQ ID NO: 237, SEQ ID NO: 238, SEQ ID NO: 239, SEQ ID NO: 240, SEQ ID NO: 243, SEQ ID NO: 244, SEQ ID NO: 245, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 264, SEQ ID NO: 282, SEQ ID NO: 284, SEQ ID NO: 285, SEQ ID NO: 286, SEQ ID NO: 287, SEQ ID NO: 289, SEQ ID NO: 290, SEQ ID NO: 291, SEQ ID NO: 291, SEQ ID NO: 292, SEQ ID NO: 293, SEQ ID NO: 295, SEQ ID NO: 300, SEQ ID NO: 310, SEQ ID NO: 313, SEQ ID NO: 314, SEQ ID NO: 316, SEQ ID NO: 317, SEQ ID NO: 320, SEQ ID NO: 330, SEQ ID NO: 331, SEQ ID NO: 336, SEQ ID NO: 343, SEQ ID NO: 376, SEQ ID NO: 377, SEQ ID NO: 378, SEQ ID NO: 379, SEQ ID NO: 380, SEQ ID NO: 381, SEQ ID NO: 382, and SEQ ID NO: 383.

Another aspect of the present invention provides a conjugate in which the antisense compound is covalently linked to at least one non-nucleotide moiety.

According to one embodiment, the non-nucleotide moiety may comprise a protein, a fatty acid chain, a sugar residue, a glycoprotein, a polymer, or any combinations thereof.

Still another aspect of the present invention provides a pharmaceutical composition for preventing or treating cancer comprising the antisense compound or the conjugate as an active ingredient.

According to one embodiment, the cancer may be selected from the group consisting of gastric cancer, esophageal cancer, bile duct cancer, ovarian cancer, cervical cancer, head and neck cancer, brain tumor, lung cancer, liver cancer, thyroid cancer, prostate cancer, bladder cancer, kidney cancer, gallbladder cancer, colorectal cancer, and pancreatic cancer.

### Advantageous Effects

Antisense compounds that modulate expression of WFDC2 according to the present invention can exhibit anticancer effects against various cancer types.

### Brief Description of Drawings

FIG. 1 is a graph showing the cancer growth inhibitory effect (cancer cell size) of subcutaneous administration of an antisense compound according to one embodiment in SNU638 cell line xenograft mouse model.
FIG. 2 is a graph showing the cancer growth inhibitory effect (cancer cell size) of intravenous administration of an antisense compound according to one embodiment in SNU638 cell line xenograft mouse model.
FIG. 3 is a graph showing the cancer growth inhibitory effect (cancer cell weight) of subcutaneous or intravenous administration of an antisense compound according to one embodiment in SNU638 cell line xenograft mouse model.
FIG. 4 depicts photographs showing the cancer growth inhibitory effect of subcutaneous or intravenous administration of an antisense compound according to one embodiment in SNU638 cell line xenograft mouse model.
FIG. 5 is a graph showing the cancer growth inhibitory effect (cancer cell size) of intravenous administration of an antisense compound according to one embodiment in SF268 cell line xenograft mouse model.
FIG. 6 depicts photographs showing the cancer growth inhibitory effect of intravenous administration of an antisense compound according to one embodiment in SF268 cell line xenograft mouse model.

### Best Mode

One aspect of the present invention is intended to provide an antisense compound comprising a modified oligonucleotide that is complementary to a nucleotide sequence in a transcript of a gene encoding WFDC2 (WAP Four-Disulfide Core Domain 2) and consists of 10 to 30 linked nucleosides.

### WFDC2 (WAP Four-Disulfide Core Domain 2)

The WFDC2 gene product is a member of the family of WAP 4-disulfide core proteins. WFDC2 is a secreted and glycosylated protein that was first observed in human epididymis tissue, and is known to be overexpressed in certain cancers, including ovarian cancer. Overexpression of WFDC2 in cancer cells suggests that this protein and its various isoforms can be a biomarker for detecting cancer and for identifying patients having a high likelihood of having cancer.

### Antisense Compound

In the present specification, "nucleotide" refers to the monomer of nucleic acid, which is composed of a combination of a nucleobase, a sugar moiety, and a phosphate group. The nucleotides can be unmodified or modified at the nucleobase, sugar moiety and/or phosphate group, and may be interpreted to encompass all nucleotide analogs, modified nucleotides, non-natural nucleotides, non-standard nucleotides, etc.

In the present specification, "nucleoside" is a glycosylamine considered to be the part of a nucleotide excluding the phosphate group and refers to a monomeric molecule consisting of a nucleobase and a sugar moiety. Like nucleotides, the nucleosides can be interpreted to encompass all nucleosides unmodified or modified at the nucleobase "G" or the sugar moiety.

In the present specification, "oligonucleotide" refers to an oligomer or polymer of ribonucleic acid (RNA) or deoxyribonucleic acid (DNA) or mimetics thereof. The oligonucleotides generally include oligonucleotides composed of covalent bonds between naturally-occurring nucleobases, sugars and nucleoside (backbone), as well as modified or substituted oligonucleotides composed of nucleotide analogs, modified nucleotides, non-natural nucleotides, or non-standard nucleotides, which function similarly. Such modified or substituted oligonucleotides have enhanced cellular uptake, enhanced affinity for nucleic acid target, and increased stability over unmodified or unsubstituted oligonucleotides in the presence of nucleases.

In the present specification, "antisense compound" is interpreted to encompass oligonucleotide capable of hybridizing with a target nucleic acid sequence by hydrogen bonding. Antisense compounds include, but are not limited to, oligonucleotides, oligonucleotide analogs, oligonucleotide mimetics, antisense oligonucleotides, siRNA, single-stranded siRNA (ss siRNA), short hairpin RNA (shRNA), microRNA mimics, ribozymes, external guide sequence oligonucleotides, and other oligonucleotides that can hybridize to target nucleic acid sequence and modulate its expression. The antisense compound is interpreted to encompass single-stranded and double-stranded oligonucleotides.

According to one embodiment, when the antisense compound is written in the 5' to 3' direction, it has a nucleotide sequence that includes the reverse complement of the target site of the target nucleic acid sequence. Preferably, the antisense compound may be complementary to a nucleotide sequence in the transcript of the gene encoding WFDC2. The transcript of the gene encoding WFDC2 is a nucleic acid that is targeted by the antisense compound, and it may be selected from an mRNA and a pre-mRNA including introns, exons and untranslated regions.

According to one embodiment, the nucleotide sequence of the transcript of the gene encoding WFDC2 is SEQ ID NO: 1 or SEQ ID NO: 2. The nucleotide sequence of SEQ ID NO: 1 is the human WFDC2 genome sequence (the complement of GenBank accession number NC_000020.11 (nucleotides 45469753 to 45481532), pre-mRNA sequence), and SEQ ID NO: 2 is the human WFDC2 mRNA sequence (RefSeq or GenBank accession number NM_006103.4).

According to one embodiment, the antisense compound may comprise a modified oligonucleotide that is at least 70%, at least 80%, at least 90% or fully complementary to any sequence of SEQ ID NO: 1 or SEQ ID NO: 2, which is the nucleotide sequence in the transcript of the gene encoding WFDC2, wherein the a modified oligonucleotide comprises at least 8 contiguous nucleobases fully complementary to any sequence of SEQ ID NO: 1 or SEQ ID NO: 2 and consists of 10 to 30, preferably 12 to 25, more preferably 14 to 23, most preferably 16 to 20 linked nucleosides.

According to one embodiment, the antisense compound may comprise a modified oligonucleotide having a nucleotide sequence that is at least 70%, at least 80%, at least 90% or fully complementary to any sequence of SEQ ID NO: 1 or SEQ ID NO: 2, which is the nucleotide sequence in the transcript of the gene encoding WFDC2, wherein the modified oligonucleotide comprises a portion of any one of SEQ ID NOs: 7 to 386 and consists of 10 to 30 linked nucleosides.

According to one embodiment, the antisense compound may have a nucleotide sequence that is at least 70%, at least 80%, at least 90% or fully complementary to any sequence of SEQ ID NO: 1 or SEQ ID NO: 2, and the antisense compound may comprise a modified oligonucleotide having a nucleotide sequence comprising at least 8 contiguous nucleobases fully complementary to any portion of an oligonucleotide sequence selected from the group consisting of start site 25 to stop site 46, start site 284 to stop site 305, start site 520 to stop site 545, start site 2222 to stop site 2344, start site 7334 to stop site 9301, start site 9506 to stop site 9551, start site 9733 to stop site 10143, start site 10271 to stop site 10302, start site 10360 to stop site 10905, start site 10977 to stop site 11292, start site 11448 to stop site 11563, and start site 11633 to stop site 11773 of the nucleotide sequence of SEQ ID NO: 1.

According to one embodiment, the antisense compound may have a nucleotide sequence that is fully complementary to any sequence in the nucleotide sequence in the transcript of the gene encoding WFDC2, and the antisense compound may comprise a modified oligonucleotide comprising at least 8 contiguous nucleobases fully complementary to any sequence in the nucleotide sequence of any one of SEQ ID NOs: 7 to 386 and consisting of 10 to 30 linked nucleosides.

According to one embodiment, the antisense compound may have a nucleotide sequence that is at least 70%, at least 80%, at least 90% or fully complementary to any sequence of SEQ ID NO: 1 or SEQ ID NO: 2, and it may comprise a modified oligonucleotide having a nucleotide sequence comprising at least 8 contiguous nucleobases that perfectly match any one oligonucleotide sequence selected from the group consisting of SEQ ID NO: 7, SEQ ID NO: 8, SEQ ID NO: 9, SEQ ID NO: 20, SEQ ID NO: 38, SEQ ID NO: 39, SEQ ID NO: 42, SEQ ID NO: 43, SEQ ID NO: 46, SEQ ID NO: 47, SEQ ID NO: 48, SEQ ID NO: 49, SEQ ID NO: 65, SEQ ID NO: 83, SEQ ID NO: 86, SEQ ID NO: 89, SEQ ID NO: 109, SEQ ID NO: 113, SEQ ID NO: 119, SEQ ID NO: 120, SEQ ID NO: 121, SEQ ID NO: 122, SEQ ID NO: 123, SEQ ID NO: 129, SEQ ID NO: 131, SEQ ID NO: 135, SEQ ID NO: 136, SEQ ID NO: 140, SEQ ID NO: 141, SEQ ID NO: 142, SEQ ID NO: 148, SEQ ID NO: 154, SEQ ID NO: 155, SEQ ID NO: 156, SEQ ID NO: 157, SEQ ID NO: 165, SEQ ID NO: 169, SEQ ID NO: 176, SEQ ID NO: 191, SEQ ID NO: 205, SEQ ID NO: 210, SEQ ID NO: 213, SEQ ID NO: 214, SEQ ID NO: 215, SEQ ID NO: 218, SEQ ID NO: 228, SEQ ID NO: 229, SEQ ID NO: 237, SEQ ID NO: 238, SEQ ID NO: 239, SEQ ID NO: 240, SEQ ID NO: 243, SEQ ID NO: 244, SEQ ID NO: 245, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 264, SEQ ID NO: 282, SEQ ID NO: 284, SEQ ID NO: 285, SEQ ID NO: 286, SEQ ID NO: 287, SEQ ID NO: 289, SEQ ID NO: 290, SEQ ID NO: 291, SEQ ID NO: 291, SEQ ID NO: 292, SEQ ID NO: 293, SEQ ID NO: 295, SEQ ID NO: 300, SEQ ID NO: 310, SEQ ID NO: 313, SEQ ID NO: 314, SEQ ID NO: 316, SEQ ID NO: 317, SEQ ID NO: 320, SEQ ID NO: 330, SEQ ID NO: 331, SEQ ID NO: 336, SEQ ID NO: 343, SEQ ID NO: 376, SEQ ID NO: 377, SEQ ID NO: 378, SEQ ID NO: 379, SEQ ID NO: 380, SEQ ID NO: 381, SEQ ID NO: 382, and SEQ ID NO: 383.

According to one embodiment, the antisense compound may comprise a modified oligonucleotide consisting of any one of the nucleotide sequences of SEQ ID NOs: 7 to 386.

According to one embodiment, the antisense compound may be a modified oligonucleotide having the nucleotide sequence of any one selected from the group consisting of SEQ ID NO: 7, SEQ ID NO: 8, SEQ ID NO: 9, SEQ ID NO: 20, SEQ ID NO: 38, SEQ ID NO: 39, SEQ ID NO: 42, SEQ ID NO: 43, SEQ ID NO: 46, SEQ ID NO: 47, SEQ ID NO: 48, SEQ ID NO: 49, SEQ ID NO: 65, SEQ ID NO: 83, SEQ ID NO: 86, SEQ ID NO: 89, SEQ ID NO: 109, SEQ ID NO: 113, SEQ ID NO: 119, SEQ ID NO: 120, SEQ ID NO: 121, SEQ ID NO: 122, SEQ ID NO: 123, SEQ ID NO: 129, SEQ ID NO: 131, SEQ ID NO: 135, SEQ ID NO: 136, SEQ ID NO: 140, SEQ ID NO: 141, SEQ ID NO: 142, SEQ ID NO: 148, SEQ ID NO: 154, SEQ ID NO: 155, SEQ ID NO: 156, SEQ ID NO: 157, SEQ ID NO: 165, SEQ ID NO: 169, SEQ ID NO: 176, SEQ ID NO: 191, SEQ ID NO: 205, SEQ ID NO: 210, SEQ ID NO: 213, SEQ ID NO: 214, SEQ ID NO: 215, SEQ ID NO: 218, SEQ ID NO: 228, SEQ ID NO: 229, SEQ ID NO: 237, SEQ ID NO: 238, SEQ ID NO: 239, SEQ ID NO: 240, SEQ ID NO: 243, SEQ ID NO: 244, SEQ ID NO: 245, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 264, SEQ ID NO: 282, SEQ ID NO: 284, SEQ ID NO: 285, SEQ ID NO: 286, SEQ ID NO: 287, SEQ ID NO: 289, SEQ ID NO: 290, SEQ ID NO: 291, SEQ ID NO: 291, SEQ ID NO: 292, SEQ ID NO: 293, SEQ ID NO: 295, SEQ ID NO: 300, SEQ ID NO: 310, SEQ ID NO: 313, SEQ ID NO: 314, SEQ ID NO: 316, SEQ ID NO: 317, SEQ ID NO: 320, SEQ ID NO: 330, SEQ ID NO: 331, SEQ ID NO: 336, SEQ ID NO: 343, SEQ ID NO: 376, SEQ ID NO: 377, SEQ ID NO: 378, SEQ ID NO: 379, SEQ ID NO: 380, SEQ ID NO: 381, SEQ ID NO: 382, and SEQ ID NO: 383.

According to one embodiment, the antisense compound that may be complementary to a nucleotide sequence in the transcript of the gene encoding WFDC2 may comprise a modified oligonucleotide that is 10 to 30 linked nucleosides in length. Preferably, the antisense compound may consist of a modified oligonucleotide that is 12 to 28, 15 to 25, 18 to 24, 19 to 22, or 20 linked nucleosides in length. Preferably, the antisense compound may be a modified oligonucleotide that is 10, 11, 12, 13, 14, 15, 16, 17, 18, 19, 20, 21, 22, 23, 24, 25, 26, 27, 28, 29, or 30 linked nucleosides in length.

According to one embodiment, the antisense compound may be single-stranded or double-stranded. When the antisense compound is double-stranded, the double strand may comprise a first modified oligonucleotide having a region complementary to a target nucleic acid and a second modified oligonucleotide having a region complementary to the first modified oligonucleotide.

### Hybridization

The antisense compound is able to select at least one target site from a nucleotide sequence in the transcript of the gene encoding WFDC2, select an oligonucleotide sufficiently complementary to the target site, and hybridize sufficiently specifically with the target site, thereby achieving a desired effect on the modulation of expression of WFDC2.

In the present specification, "hybridization" refers to hydrogen bonding that may be Watson-Crick, Hoogsteen or reversed Hoogsteen hydrogen bonding between complementary nucleosides or nucleobases. For example, adenine and thymine are complementary nucleobases that pair through the formation of hydrogen bonds.

In the present invention, "hybridizable" or "complementary" or "substantially complementary" means that a nucleic acid (e.g., RNA or DNA) comprises a sequence of nucleotides that enables it to non-covalently bind, i.e., form adenine (A) pairing with thymidine (T), adenine (A) pairing with uracil (U), and guanine (G) pairing with cytosine (C), "anneal", or "hybridize," to another nucleic acid in a sequence-specific, antiparallel manner (i.e., a nucleic acid specifically binds to a complementary nucleic acid) under the appropriate *in vitro* and/or in *vivo* conditions of temperature and solution ionic strength.

According to one embodiment, the hybridization occurs between the antisense compound disclosed herein and a nucleotide sequence in the transcript of the gene encoding WFDC2. The most common mechanism of hybridization involves hydrogen bonding between complementary nucleobases of nucleic acid molecules.

Hybridization can occur under varying conditions. Stringent conditions are sequence-dependent and are determined by the nature and composition of the nucleic acid molecules to be hybridized. Methods of determining whether a sequence is specifically hybridizable to a target nucleic acid are well known in the art.

### Complementarity

In the present specification, the term "complementary" refers to the capacity for precise pairing between two nucleotides. For example, if the nucleotide sequences of two different nucleic acids or oligonucleotides are written in the 5' to 3' direction, when the nucleotide sequence of a certain portion of one nucleic acid or oligonucleotide is aligned in the opposite direction, it non-covalently binds, i.e., forms adenine (A) pairing with thymidine (T), adenine (A) pairing with uracil (U), and guanine (G) pairing with cytosine (C), to a certain portion of the remaining one nucleic acid or oligonucleotide, and in this case, the two nucleic acids or oligonucleotides are referred to as complementary.

Thus, "specifically hybridizable" and "complementary" may be interpreted as terms which are used to indicate a sufficient degree of complementarity or precise pairing such that stable and specific binding can occur between the oligonucleotide and the DNA or RNA target. It is known in the art that the sequence of an antisense compound need not be 100% complementary to that of its target nucleic acid to be specifically hybridizable.

The antisense compound is specifically hybridizable to the target DNA or RNA and can interfere with the normal function of the target DNA or RNA, and it is interpreted that there is a sufficient degree of complementarity to avoid non-specific binding of the antisense compound to non-target sequences under conditions in which specific binding is desired, i.e., under physiological conditions in the case of *in vivo* assays or therapeutic treatment, and in the case of *in vitro* assays, under conditions in which the assays are performed.

Non-complementary nucleobases between the antisense compound and a target nucleic acid may be tolerated provided that the antisense compound is able to specifically hybridize to the target nucleic acid. Moreover, the antisense compound may hybridize with one or more segments of a nucleic acid such that intervening or adjacent segments are not involved in the hybridization event (e.g., a loop structure, mismatch or hairpin structure).

According to one embodiment, the antisense compound of the present invention or the modified oligonucleotide constituting the antisense compound may be at least 70%, at least 75%, at least 80%, at least 85%, at least 90%, at least 95%, at least 96%, at least 97%, at least 98% or at least 99% complementary to a nucleotide sequence in the transcript of the gene encoding WFDC2 (for example, SEQ ID NO: 1 or SEQ ID NO: 2). Percent complementarity of the antisense compound with a target nucleic acid may be determined using routine methods known in the art. For example, an antisense compound in which 18 of 20 nucleobases of the antisense compound are complementary to a target region can specifically hybridize, and represents 90% complementarity. In this example, the remaining non-complementary nucleobases may be clustered or interspersed with complementary nucleobases and need not be contiguous to each other or to complementary nucleobases. As such, an antisense compound which is 18 nucleobases in length having 4 non-complementary nucleobases which are flanked by two regions of complete complementarity with the target nucleic acid has 77.8% overall complementarity with the target nucleic acid, and thus is interpreted to fall within the scope of the present invention. Percent complementarity of the antisense compound with a region of a target nucleic acid can be determined routinely using BLAST programs and PowerBLAST programs known in the art (Altschul et al., J. Mol. Biol., 1990, 215, 403 410; Zhang and Madden, Genome Res., 1997, 7, 649 656). Meanwhile, percent homology, sequence identity or complementarity can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

According to one embodiment, the antisense compound of the present invention or the modified oligonucleotide constituting the antisense compound may be at least 80%, preferably at least 90%, most preferably fully complementary (100% complementary) to a nucleotide sequence in the transcript of the gene encoding WFDC2 (for example, SEQ ID NO: 1 or SEQ ID NO: 2). In the present specification, "fully complementary" means that each nucleobase of the antisense compound is capable of precise base pairing with the corresponding nucleobases of a target nucleic acid.

According to one embodiment, the location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e., linked) or non-contiguous. According to one embodiment, the non-complementary nucleobase may be located in the wing segment of a gapmer antisense oligonucleotide.

According to one embodiment, the antisense compound of the present invention may comprise those which are complementary to a nucleotide sequence portion in the transcript of the gene encoding WFDC2. In the present specification, "portion" refers to a defined number of contiguous (i.e., linked) nucleobases within a region or segment of a target nucleic acid. The portion can also refer to a defined number of contiguous nucleobases of the antisense compound. According to one embodiment, the antisense compound may be complementary to at least an 8-nucleobase portion, at least a 12-nucleobase portion, or at least a 15-nucleobase portion of a target segment. Antisense compounds that are complementary to at least a 9, 10, 11, 12, 13, 14, 15, 16, 17, 18, 19, 20, or more nucleobase portion of a target segment, or a nucleobase portion within a range defined by any two of these values, are also interpreted to be included in the above range.

### Modified Oligonucleotide

According to one embodiment of the present invention, the antisense compound may comprise a modified oligonucleotide, wherein the modified oligonucleotide may comprise at least one modification selected from at least one modified internucleoside linkage, at least one modified nucleoside comprising a modified sugar moiety, and at least one modified nucleoside comprising a modified nucleobase.

### Modification of Sugar Moiety

According to one embodiment, the modified nucleoside may be a modified nucleoside comprising a non-bicyclic modified sugar moiety, and/or a bicyclic or tricyclic sugar moiety, and/or a sugar moiety modified with a sugar surrogate or sugar mimetic, etc.

According to one embodiment, the modified nucleoside may comprise a sugar moiety substituted with at least one substitute selected from the group consisting of 2'-O-alkyl such as 2'-O-methyl, 2'-O-alkoxyalkyl such as 2'-O-methoxyethyl, 2'-amino, 2'-allyl, 2'-fluoro, 2'-arabino-fluoro, 2'-O-N-substituted acetamide such as 2'-OCH₂C(=O)-NHCH₃(NMA), 2'-O-benzyl and 2'-O-methyl-4-pyridine, 4'-O-methyl, 5'-methyl, 5'-vinyl, and 5'-methoxy, without being limited thereto.

The antisense compound according to one embodiment of the present invention may comprise at least one modified nucleoside having a sugar moiety optionally substituted or modified. Modification of the sugar moiety imparts nuclease stability, binding affinity or some other beneficial biological properties to the antisense compound. The (pento)furanosyl sugar ring of the natural nucleoside can be modified in a number of ways including, but not limited to: addition of a substituent group, particularly at the 2' position; bridging of two non-geminal ring atoms to form a bicyclic nucleic acid (BNA); and substitution of an atom or group such as -S-, -N(R)- or - C(R1)(R2) for the ring oxygen at the 4'-position. Modified sugar moieties include, but are not limited to, substituted sugars, especially 2'-substituted sugars having a 2'-F, 2'-OCH₂(2'-OMe) or a 2'-O(CH₂)₂-OCH₃ (2'-O-methoxyethyl or 2'-MOE) substituent group; and bicyclic modified sugars (BNAs), having a 4'-(CH₂)n-O-2' bridge, where n=1 or n=2. Methods for the preparation of modified sugars are well known skilled in the art. The base moiety in the nucleoside comprising the modified sugar moiety may remain to hybridize with the target nucleic acid.

According to one embodiment, the modified nucleoside comprises one of the following at the 2' position: F; O-, S-, or N-alkyl; O-, S- or N-alkenyl; O-, S- or N-alkynyl; O-alkyl-O-alkyl; O-alkyl-O-alkyl-N (dialkyl); or O-alkyl-carboxylamide, wherein the alkyl, alkenyl and alkynyl are substituted or unsubstituted C₁ to C₁₀ alkyl or C₂ to C₁₀ alkenyl and alkynyl. Particularly preferred are O[(CH₂)nO]m CH₃, O(CH₂)nOCH₃, O(CH₂)nNH₂, O(CH₂)nCH₃, O(CH₂)nONH₂, O(CH₂)nO(CH₂)nN[(CH₂)mCH₃]₂, O(CH₂)nC(=O)-NHCH₃, and O(CH₂)nON[(CH₂)mCH₃]₂, where n and m are from 0 to 10. Other preferred modified oligonucleotides comprise one of the following at the 2' position: C₁ to C₁₀ lower alkyl, substituted lower alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, or polyalkylamino substituents. Preferably, the modification may include 2'-methoxyethoxy (2'-O-CH₂CH₂OCH₃, also known as 2'-O-(2-methoxyethyl) or 2'-MOE) (Martin et al., Helv. Chim. Acta, 1995, 78, 486-504), i.e., an alkoxyalkoxy group. The modification may include 2'-dimethylaminooxyethoxy, i.e., a (CH₂)₂ON(CH₃)₂ group, also known as 2'-DMAOE, and 2'-dimethylaminoethoxyethoxy, also known as 2'-O-dimethylaminoethoxyethyl or 2'-DMAEOE), i.e., 2'-O(CH₂)₂O(CH₂)₂--N(CH₃)₂.

Other preferred modifications may include 2'-methoxy (2'-O-CH₃), 2'-aminopropoxy (2'-OCH₂CH₂CH₂NH₂), 2'-allyl (2'-CH₂-CH=CH₂), 2'-O-allyl (2'-O-CH₂-CH=CH₂) and 2'-fluoro (2'-F). The 2'-modification may be at the arabino (up) position or ribo (down) position. A preferred 2'-arabino modification is 2'-F. Similar modifications may also be made at other positions on the nucleoside, particularly the 3' position of the sugar on the 3' terminal nucleoside or the 5' position of 5' terminal nucleoside.

The bicyclic or tricyclic sugar moiety may be, for example, selected from the group consisting of locked nucleic acid (LNA), constrained ethyl bicyclic nucleic acid (cEt), 2'-O,4'-C-ethylene-bridged nucleic acid (ENA), and tricyclo-DNA, without being limited thereto.

According to one embodiment, the modified nucleoside may comprise a sugar surrogate having a 6-membered ring or an acyclic moiety. The sugar surrogate may be selected from the group consisting of morpholino rings such as phosphorodiamidate morpholino oligomer (PMO), cyclohexenyl rings, cyclohexyl rings, and tetrahydropyranyl rings such as hexitol, anitol, mannitol, and fluoro hexitol, without being limited thereto. Various other bicyclo and tricyclo sugar surrogate ring systems that may be used to modify nucleosides for incorporation into the antisense compound according to the invention are known in the art. Such ring systems can undergo various substitutions to enhance activity.

In addition, the sugar surrogate may be, for example, an acyclic moiety such as unlocked nucleic acid (UNA) or peptide nucleic acid (PNA), without being limited thereto.

Peptide nucleic acid (PNA) is a type of nucleic acid analogue in which the nucleobases are linked via peptide bonds rather than phosphate bonds, and the phosphodiester bonds are replaced by peptide bonds. PNA has nucleobases such as adenine, thymine, guanine, and cytosine, and thus can specifically hybridize with nucleic acids. PNA is not found in nature, but is artificially synthesized by a chemical method. PNA can form a double strand by hybridization with a nucleic acid having a complementary base sequence. In addition, PNA is characterized in that it is not only chemically stable because it is electrically neutral, but also biologically stable because it is not degraded by nucleases or proteases. PNA having an N-aminoethyl glycine backbone is most widely used, but as is known in the art, PNA with a modified backbone may also be used (P.E. Nielsen and M. Egholm "An Introduction to PNA" in P.E. Nielsen (Ed.) "Peptide Nucleic Acids: Protocols and Applications" 2nd Ed. Page 9 (Horizon Bioscience, 2004)).

Unlocked nucleic acid (UNA) is a modified nucleoside that does not have the C2'-C3' bond of ribose. Due to the open chain structure, the steric configuration is not restricted, and the oligonucleotide flexibility can be adjusted. It is known that when UNA is included in an antisense oligonucleotide, it can lower the Tm value by about 5°C to 10°C and reduce off-targets.

### Modification of Nucleobases

According to one embodiment, the modified nucleoside may be a modified nucleoside comprising at least one modified nucleobase selected from the group consisting of pseudouridine, 2'-thiouridine, N6'-methyladenosine, 5'-methylcytidine, 5'-fluoro-2-deoxyuridine, N-ethylpiperidine 7'-EAA triazol modified adenine, N-ethylpiperidine 6'-triazol modified adenine, 6'-phenylpyrrolocytosine, 2',4'-difluorotoluylribonuleoside, and 5'-nitroindole.

Unmodified or natural nucleobases mean the purine bases adenine (A) and guanine (G), and the pyrimidine bases thymine (T), cytosine (C) and uracil (U).

The modified nucleoside may also include nucleobase modifications or substitutions. Nucleobase modifications or substitutions are structurally distinct forms, but are functionally interchangeable with naturally occurring or synthetic unmodified nucleobases. Both naturally occurring and modified nucleobases are capable of participating in hydrogen bonding. Such nucleobase modifications impart nuclease stability, binding affinity or some other beneficial biological properties to the antisense compound. For example, certain nucleobase substitutions, such as 5-methylcytosine substitutions, are known to increase nucleic acid duplex stability by 0.6 to 1.2°C, and thus may be particularly useful for increasing the binding affinity of the antisense compound for a target nucleic acid.

For example, the modified nucleobases include, but are not limited to, 5'-hydroxymethyl cytosine, xanthine, hypoxanthine, 2'-aminoadenine, 6'-methyl and other alkyl derivatives of adenine and guanine, 2'-propyl and other alkyl derivatives of adenine and guanine, 2'-thiouracil, 2'-thiothymine and 2'-thiocytosine, 5'-halouracil and cytosine, 5'-propynyl (-C≡C-CH₃) uracil and cytosine and other alkynyl derivatives of pyrimidine bases, 6'-azo uracil, cytosine and thymine, 5'-uracil (pseudouracil), 4'-thiouracil, 8'-halo, 8'-amino, 8'-thiol, 8'-thioalkyl, 8'-hydroxyl and other 8'-substituted adenines and guanines, 5'-halo (particularly 5'-bromo), 5'-trifluoromethyl and other 5'-substituted uracils and cytosines, 7'-methylguanine and 7'-methyladenine, 2'-F-adenine, 2'-amino-adenine, 8'-azaguanine and 8'-azaadenine, 7'-deazaguanine and 7'-deazaadenine and 3-deazaguanine and 3'-deazaadenine, tricyclic pyrimidines such as phenoxazine cytidine (1H-pyrimido[5,4-b][1,4]benzoxazin-2(3H)-one), phenothiazine cytidine (1H-pyrimido[5,4-b] [1,4]benzothiazin-2(3H)-one), and G-clamps such as a substituted phenoxazine cytidine (e.g., 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one).

Heterocyclic base moieties may also include those in which the purine or pyrimidine base is replaced with other heterocycles, for example, 7'-deaza-adenine, 7'-deazaguanosine, 2'-aminopyridine and 2'-pyridone. Nucleobases that are particularly useful for increasing the binding affinity of the antisense compounds include, but are not limited to, 5'-substituted pyrimidines, 6'-azapyrimidines and N-2, N-6 and O-6 substituted purines, including 2'-aminopropyladenine, 5'-propynyluracil and 5'-propynylcytosine. In addition, the modified nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, Englisch et al., Angewandte Chemie, International Edition, 1991, 30, 613, and those disclosed in Sanghvi, Y. S., Chapter 15, Antisense Research and Applications, pages 289-302, Crooke, S. T. and Lebleu, B. ed., CRC Press, 1993.

### Modified Internucleoside Linkages

According to one embodiment, the modified internucleoside linkage in the antisense compound may be at least one modified internucleoside linkage selected from the group consisting of phosphotriester, phosphoramidate, mesyl phosphoramidate, phosphorothioate, phosphorodithioate, methylphosphonate, and methoxypropyl-phosphonate.

As is known in the art, a nucleoside is a combination of a nucleobase and a sugar moiety. A nucleotide further comprises a phosphate group covalently linked to the sugar moiety of the nucleoside. For nucleotides including a pentofuranosyl sugar, the phosphate group may be linked to the 2', 3' or 5' hydroxyl moiety of the sugar. In forming oligonucleotides, the phosphate groups covalently link adjacent nucleosides to one another to form a linear polymeric compound. In turn, the respective ends of this linear polymeric structure can be further joined to form a circular structure, but open linear structures are generally preferred. Within the oligonucleotide structure, the phosphate groups are commonly referred to as forming the internucleoside backbone of the oligonucleotide. The naturally occurring linkage or backbone of RNA and DNA is a 3' to 5' phosphodiester linkage. The antisense compounds according to one embodiment may comprise at least one modified internucleoside linkage in addition to naturally occurring internucleoside linkages, and such compounds are often selected over antisense compounds having naturally occurring internucleoside linkages because of desirable properties such as enhanced cellular uptake, enhanced affinity for target nucleic acids, and increased stability in the presence of nucleases.

One specific example of a preferred antisense compound that may be used in the present invention is an oligonucleotide containing a modified backbone or non-natural internucleoside linkage. As defined above, oligonucleotides having modified backbones include those that retain a phosphorus atom in the backbone and those that do not have a phosphorus atom in the backbone. In addition, for the purposes of the present specification, and as referenced in the art, modified oligonucleotides that do not have a phosphorus atom in their internucleoside backbone are also considered to be oligonucleotides.

Modified internucleoside linkages in the antisense compounds according to the present invention may include internucleoside linkages that retain a phosphate as well as internucleoside linkages that do not have a phosphate. Representative phosphate-containing internucleoside linkages include, but are not limited to, phosphorothioates, chiral phosphorothioates, phosphorodithioates, phosphotriesters, aminoalkyl phosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkyl phosphoramidates, mesyl phosphoramidates, thiono-phosphoramidates, thionoalkylphosphonates, thionoalkylphospho-triesters, selenophosphates and boranophosphates having normal 3'-5' linkages, 2'-5' linked analogs of these, and those having inverted polarity wherein one or more internucleotide linkages are a 3' to 3', 5' to 5' or 2' to 2' linkage. In addition, oligonucleotides having inverted polarity comprise a single 3' to 3' linkage at the 3'-most internucleotide linkage, i.e., a single inverted nucleoside residue which may be abasic (the nucleobase is missing or has a hydroxyl group in place thereof). Various salts, mixed salts and free acid forms may also be included.

Preferred modified oligonucleotide backbones that do not include phosphorus atom therein may be backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include, but are not limited to, backbones having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene-containing backbones; sulfamate backbones; methyleneimino and methylenehydrazino backbones; sulfonate and sulfonamide backbones; amide backbones; and backbones having mixed N, O, S and CH₂ component parts. Methods for preparing such phosphate-containing internucleoside linkages and phosphate-free internucleoside linkages are known in the art.

In other embodiment, the hydroxyl group at the 5' end of the antisense compound may be substituted with one selected from the group consisting of 5'-(E)-vinylphosphonate, 5'-methylphosphonate, (S)-5'-C-methyl with phosphate, and 5'-phosphorothioate. It is known that this modified antisense compound is well loaded into an RNA-induced silencing complex (RISC) and functions as single-stranded short interfering RNA (ss siRNA) or double-stranded short interfering RNA (ds siRNA).

### Antisense Compound Motif

According to one embodiment, the antisense compound has a chimeric form of Lx - Dy - Lz, where L may be a modified nucleoside. Here, D is DNA, x and z are any integers ranging from 1 to 7, which may be the same as or different from each other, and y is any integer ranging from 5 to 25. Preferably, x and z may be any integers ranging from 1 to 5, y may be any integer ranging from 7 to 24. More preferably, x and z may be any integers ranging from 3 to 5, and y may be any integer ranging from 8 to 23. At least the sugar moiety of the L region closest to the D region may be modified, so that the boundary between the L region and the D region can be defined. In addition, in all of the above regions (L regions and D region), each internucleoside linkage may include a phosphodiester or one or more of the above-described modified internucleoside linkages (e.g., phosphorothioates), and the nucleobase in the nucleoside may also include one or more of natural nucleobases or the above-described modified nucleobases.

According to one embodiment, the modified oligonucleotide may comprise a gap segment consisting of linked deoxynucleosides, a 5' wing segment consisting of linked nucleosides, and a 3' wing segment consisting of linked nucleosides, wherein the gap segment may be positioned between the 5' wing segment and the 3' wing segment and wherein the nucleoside of each wing segment may comprise a modified sugar moiety or a sugar surrogate.

Chimeric antisense compounds may typically contain at least one region modified so as to confer increased resistance to nuclease degradation, increased cellular uptake, increased binding affinity for a target nucleic acid, and/or increased inhibitory activity. Chimeric antisense compounds may be formed as composite structures of two or more oligonucleotides or modified oligonucleotides. Such compounds have also been referred to in the art as hybrids or gapmers, and the preparation of such gapmer structures is disclosed in U.S. Pat. Nos. 5,013,830; 5,149,797; 5,220,007; 5,256,775; 5,366,878; 5,403,711; 5,491,133; 5,565,350; 5,623,065; 5,652,355; 5,652,356; and 5,700,922.

In a gapmer, an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment (the D region in the antisense compound herein) supports cleavage of a target nucleic acid, while the wing segments (the L regions in the antisense compound herein) may comprise a modified oligonucleotide comprising modified nucleosides to enhance stability, affinity, and exonuclease resistance. If necessary, the gap segment may also comprise a modified oligonucleotide. The modified oligonucleotide may comprise at least one modification selected from at least one modified inteernucleoside linkage, at least one modified nucleoside comprising a modified sugar moiety, and at least one modified nucleoside comprising a modified nucleobase, and each modification is as described above.

Preferably, each distinct region in the gapmer may comprise uniform sugar moieties. Additionally, each distinct region is demarcated by a different sugar moiety, but the sugar moiety within each region may be in the form of a mixmer freely selected from unmodified nucleotides and modified nucleotides. According to one embodiment of the present invention, this wing segment-gap segment-wing segment motif can be expressed in a form such as Lx - Dy - Lz, where x represents the length of the 5' wing segment, y represents the length of the gap segment, and z represents the length of the 3' wing segment. The antisense compound according to one embodiment may have a gapmer motif. In the antisense compound according to one embodiment, x, y and z include, for example, 5-10-5, 3-10-3, 1-12-1, 2-10-3, 3-9-4, 3-8-3, 1-9-2, 2-13-5, 4-8-4, 4-12-3, 4-12-4, 3-14-3, 2-16-2, 1-18-1, 2-10-2, 1-10-1 or 2-8-2, without being limited thereto. According to another embodiment, the antisense compound may have a wing segment-gap segment or gap segment-wing segment configuration. That is, when x or z is 0, the antisense compound may have a "wingmer" motif. The wingmer structure includes, for example, 10-10, 8-10, 5-10, 8-4, 4-12, 12-4, 3-14, 16-2, 18-1, 10-3 , 2-10, 1-10 or 8-2, without being limited thereto.

In one embodiment, the features of the 3' wing segment and the features of the 5' wing segment of the antisense compound may be selected independently. Additionally, in the embodiment, the number of monomers in the 5' wing segment (x in Lx) and the number of monomers in the 3' wing segment (z in Lz) may be the same or different. In addition, the modifications, if any, in the 5' wing segment may be the same as the modifications, if any, in the 3' wing segment or such modifications, if any, may be different; and the monomeric linkages in the 5' wing segment and the monomeric linkages in the 3' wing segment may be the same or different. That is, all of the regions do not have to be uniformly modified, and one or more of the modifications may be introduced into one or more nucleotides in the antisense oligonucleotide.

### Conjugate

Another aspect of the present invention provides a conjugate in which the antisense compound is covalently linked to at least one non-nucleotide moiety. According to one embodiment, the non-nucleotide moiety may comprise a protein, a fatty acid chain, a sugar residue, a glycoprotein, a polymer, or any combinations thereof.

In the present specification, "conjugate" refers to an antisense compound or antisense oligonucleotide covalently linked to a non-nucleotide moiety (conjugate moiety or region C or third region). This conjugation may improve the pharmacology of the antisense oligonucleotide, for example, by affecting the activity, cellular distribution, cellular uptake or stability of the antisense oligonucleotide. According to one embodiment, the non-nucleotide moiety may modify or enhance the pharmacokinetic properties of the antisense oligonucleotide by improving cellular distribution, bioavailability, metabolism, excretion, permeability, and/or cellular uptake of the antisense oligonucleotide. In addition, the non-nucleotide moiety may target the antisense oligonucleotide to a specific organ, tissue or cell type and thereby enhance the effectiveness of the antisense oligonucleotide in that organ, tissue or cell type. In addition, the non-nucleotide moiety may serve to reduce the activity of the antisense oligonucleotide in non-target cell types, tissues or organs, for example, off target activity or activity in non-target cell types, tissues or organs. International Patent Publications WO93/07883 and WO2013/033230 disclose suitable non-nucleotide moieties.

According to one embodiment, the non-nucleotide moieties include, but are not limited to, intercalators, reporter molecules, polyamines, polyamides, peptides, carbohydrates, vitamin moieties, polyethylene glycols, thioethers, polyethers, cholesterols, cholic acid moieties, folate, lipids, phospholipids, biotin, phenazine, phenanthridine, anthraquinone, adamantane, acridine, fluoresceins, rhodamines, coumarins, fluorophores, and dyes.

According to one embodiment, the non-nucleotide moiety may comprise an active drug substance, for example, aspirin, warfarin, ketoprofen, carprofen, diazepine, an antibacterial agent, or an antibiotic.

According to one embodiment, the non-nucleotide moiety may further comprise an antibody.

According to one embodiment, the non-nucleotide moiety may be linked to the 5' end or 3' end of the antisense compound or antisense oligonucleotide.

According to one embodiment, the non-nucleotide moiety may comprise at least 1 to 3 N-acetylgalactosamines (GalNAc).

### Formulations

Various formulations have been developed to facilitate oligonucleotide use. For example, oligonucleotides can be delivered to a subject or a cellular environment using a formulation that minimizes degradation, facilitates delivery and/or uptake, or provides another beneficial property to the oligonucleotides in the formulation.

In one embodiment, antisense oligonucleotides for reducing expression of WFDC2 can be suitably formulated such that when administered to a subject, either into the immediate environment of a target cell or systemically, a sufficient portion of the oligonucleotides enter the cell to reduce WFDC2 expression. According to one embodiment, the antisense oligonucleotides can be formulated in buffer solutions such as phosphate-buffered saline solutions, liposomes, micellar structures, and capsids. In addition, naked oligonucleotides or conjugates thereof may be formulated in water or in an aqueous solution (e.g., water with pH adjustments) or in basic buffered aqueous solutions (e.g., PBS).

According to one embodiment, formulations of oligonucleotides with cationic lipids can be used to facilitate transfection of the oligonucleotides into cells. For example, cationic lipids, such as lipofection, cationic glycerol derivatives, and polycationic molecules (e.g., polylysine) can be used. Suitable lipids include Oligofectamine, Lipofectamine (Life Technologies), NC388 (Ribozyme Pharmaceuticals, Inc.), or FuGene 6 (Roche), all of which can be used according to the manufacturer's instructions. This formulation may comprise a lipid nanoparticle.

In addition, the formulation may comprise an excipient. The excipient comprises a liposome, a lipid, a lipid complex, a microsphere, a microparticle, a nanosphere, or a nanoparticle, or may be otherwise formulated for administration to the cells, tissues, organs, or body of a subject in need thereof (see, for example, Remington: The Science and Practice of Pharmacy, 22nd edition, Pharmaceutical Press, 2013). The excipient confers, to a composition, improved stability, improved absorption, improved solubility and/or therapeutic enhancement of the active ingredient. In addition, the excipient may be a buffering agent (e.g., sodium citrate, sodium phosphate, a tris base, or sodium hydroxide) or a vehicle (e.g., a buffered solution, petrolatum, dimethyl sulfoxide, or mineral oil).

In some embodiments, an oligonucleotide may be lyophilized for extending its shelf-life and then made into a solution before use. Accordingly, an excipient in a composition comprising the oligonucleotide constituting the antisense compound according to the present invention may be a lyoprotectant (e.g., mannitol, lactose, polyethylene glycol, or polyvinyl pyrrolidone), or a collapse temperature modifier (e.g., dextran, ficoll, or gelatin).

Pharmaceutical compositions suitable for injectable use may include sterile aqueous solutions (where water soluble) or dispersions and sterile powders for the extemporaneous preparation of sterile injectable solutions or dispersions. For intravenous or subcutaneous administration, suitable carriers may include physiological saline, bacteriostatic water, Cremophor EL.TM. (BASF) or phosphate buffered saline (PBS). Also, the carrier can be a solvent or dispersion medium containing, for example, water, ethanol, polyol (for example, glycerol, propylene glycol, and liquid polyethylene glycol, and the like), and suitable mixtures thereof. In many cases, it will be preferable to include isotonic agents, for example, sugars, polyalcohols such as mannitol, sorbitol, and sodium chloride in the composition. Sterile injectable solutions can be prepared by incorporating the oligonucleotide in a required amount in a selected solvent with one or a combination of ingredients enumerated above, as required, followed by filtered sterilization. The pharmaceutical composition may contain at least about 0.1% of the therapeutic agent (e.g., an antisense oligonucleotide for reducing WFDC2 expression) or more, although the percentage of the active ingredient may be between about 1% and about 80% of the weight or volume of the total composition. Factors such as solubility, bioavailability, biological half-life, route of administration, product shelf life, as well as other pharmacological considerations will be contemplated in the preparation of such formulations.

### Treatment Diseases and Methods

The antisense compound or conjugate comprising the same according to the present invention may be used as a cancer treatment agent as a pharmaceutical composition for preventing or treating cancer. "Cancer" refers to a group of diseases characterized by excessive cell proliferation with the ability to infiltrate surrounding tissues when the normal cell death balance is broken. "Treatment" refers to any action of alleviating, ameliorating or beneficially changing symptoms of cancer by administration of the pharmaceutical composition according to the present invention.

According to one embodiment, the cancer may be at least one selected from the group consisting of carcinomas originating from epithelial cells, such as gastric cancer, esophageal cancer, ovarian cancer, head and neck cancer, brain tumor, thyroid cancer, lung cancer, laryngeal cancer, colon/rectal cancer, liver cancer, gallbladder cancer, bile duct cancer, bladder cancer, pancreatic cancer, breast cancer, uterine cancer, cervical cancer, prostate cancer, kidney cancer, and skin cancer, sarcomas originating from connective tissue cells, such as bone cancer, muscle cancer, fat cancer, and fibrous cell cancer, blood cancer originating from hematopoietic cells, such as leukemia, lymphoma, and multiple myeloma, and tumors occurring in nervous tissue. Preferably, the cancer may be solid cancer.

Treatment methods using pharmaceutical composition according to the present invention involve administering to a subject an effective amount of the pharmaceutical composition, that is, an amount capable of producing a desirable therapeutic result. A therapeutically acceptable amount is preferably an appropriate dosage that is capable of treating a disease. The appropriate dosage will depend on certain factors, including the subject's size, body surface area, age, the particular composition to be administered, the active ingredient(s) in the composition, time and route of administration, general health, and other drugs being administered concurrently. The composition according to the present invention may be administered orally (e.g., by gastric feeding tube, by duodenal feeding tube, via gastrostomy or rectally), or parenterally (e.g., subcutaneous injection, intravenous injection or infusion, intra-arterial injection or infusion, intramuscular injection), topically (e.g., epicutaneous, inhalational, via eye drops, or through a mucous membrane), or by direct injection into a target organ (e.g., the liver of a subject). Preferably, the antisense compound according to the present invention may be administered intravenously or subcutaneously, and may be administered at a dose in a range of 0.1 mg/kg to 50 mg/kg, 0.1 mg/kg to 30 mg/kg, 0.1 mg/kg to 20 mg/kg, 0.1 mg/kg to 5 mg/kg, or 0.5 mg/kg to 5 mg/kg. In some embodiments, the subject to be treated is preferably a human or non-human primate or other mammalian subject, but may include dogs, cats, horses, cattle, pigs, sheep, goats, chickens, mice, rats, guinea pigs, or hamsters.

### Combination Therapies

According to one embodiment of the present invention, the pharmaceutical composition may be co-administered with one or more other pharmaceutical agents. According to one embodiment, the one or more other pharmaceutical agents may be designed to treat the same disease or condition as that in the subject of the present invention. Alternatively, the one or more other pharmaceutical agents may be designed to treat an undesired effect of one or more pharmaceutical compositions of the present invention, or may be co-administered with another pharmaceutical agent to treat an undesired effect of that other pharmaceutical agent.

According to one embodiment, the pharmaceutical composition of the present invention and one or more pharmaceutical agents may be administered at the same time or at different times. In addition, one or more pharmaceutical compositions of the present invention and one or more other pharmaceutical agents may be prepared together in a single formulation or prepared separately.

According to one embodiment, pharmaceutical agents that are co-administered with the pharmaceutical composition of the present invention can enhance the therapeutic effect, resulting in an excellent therapeutic effect, that is, a synergistic effect. In other words, the present invention may provide a pharmaceutical composition comprising an antisense compound and one or more pharmaceutical agents that function by a non-antisense mechanism. The pharmaceutical agents may be chemotherapeutic agents. The chemotherapeutic agents may be, for example, daunorubicin, daunomycin, dactinomycin, doxorubicin, epirubicin, idarubicin, esorubicin, bleomycin, mafosfamide, ifosfamide, cytosine arabinoside, bis-chloroethylnitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercaptopurine, 6-thioguanine, cytarabine, 5-azacytidine, hydroxyurea, deoxycoformycin, hydroxyperoxycyclo-phosphoramide, 5-fluorouracil (5-FU), 5-fluorodeoxyuridine (5-FUdR), methotrexate (MTX), colchicine, taxol, vincristine, vinblastine, etoposide (VP-16), trimetrexate, irinotecan, topotecan, gemcitabine, teniposide, cisplatin or diethylstilbestrol (DES), without being limited thereto. When used with the antisense compound of the invention, the chemotherapeutic agents may be used individually (e.g., 5-FU and oligonucleotide), sequentially (e.g., 5-FU and oligonucleotide for a period of time followed by MTX and oligonucleotide), or in combination with one or more other such chemotherapeutic agents (e.g., 5-FU, MTX and oligonucleotide, or 5-FU, radiotherapy and oligonucleotide). Anti-inflammatory drugs (including, but not limited to, nonsteroidal anti-inflammatory drugs and corticosteroids), and antiviral drugs (including, but not limited to, ribivirin, vidarabine, acyclovir and ganciclovir) may also be contained in the composition of the present invention.

### Mode for Invention

Hereinafter, one or more embodiments will be described in more detail by way of examples. However, these examples are intended to illustrate one or more embodiments and the scope of the present invention is not limited to these examples.

### Experimental Methods

### 1. Cell Culture

Human gastric cancer cell line SNU638 or glioblastoma cell line SF268 were cultured in an incubator at 37°C at a carbon dioxide concentration of 5% (v/v) using RPM1-1640 (#Sh30027.01, Hyclone) containing 10% (v/v) FBS (#SH30084.03HI, Hyclone) and 1% (v/v) antibiotic (Penicillin-Streptomycin, #LS202-02, Welgene) solution as a medium.

The PANC-1 cell line derived from pancreatic cancer epithelial tissue was cultured in Dulbecco's modified Eagle's Medium (DMEM) containing 10% FBS and 1% antibiotics (Penicillin-Streptomycin, #LS202-02, Welgene) at 37°C at a carbon dioxide concentration of 5% (v/v). Subculture was performed every 4 days, and only cells that had been subcultured 5 to 10 times were used as cells for transformation experiments.

### 2. Preparation of Antisense Oligonucleotides (ASOs)

The antisense oligonucleotides (ASOs) used in this Example were designed to target various regions in the pre-mRNA (SEQ ID NO: 1) or mRNA (SEQ ID NO: 2) of WFDC2, and were custom-made by Integrated DNA Technology or synthesized by repeatedly applying the standardized phosphoramidite chemistry cycle as shown in Table 1 below to the universal linker bound to a controlled-pore glass (CPG) solid phase support in an automated DNA synthesizer (BioAutomation model MerMade 12) or an automated peptide synthesizer (Biotage model Syro 1).

**[Table 1]**

| Cycle step | Reagent/solvent |
|---|---|
| Wash | Acetonitrile (ACN) |
| Detritylation | 3% tichloroacetic acid in dichloromethane |
| Wash | ACN |
| Coupling | 0.07M DMT-X-CE phosphoramidite (X= dA/dG/dC/dT for DNA or 2'-O-MOE-A/G/C/T or etc.) in ACN 0.25M 5-(ethylthio)-1H-tetrazole (ETT) in ACN |
| Wash | ACN |
| Oxidation | Oxidizer: 0.02M iodine/H2O/pyridine/THF |
| Wash | ACN |
| Capping | CAP A: 10% acetic anhydride in THFCAP B: 10% N-methylimidazole in pyridine-THF |
| Wash | ACN |

For the phosphorothioate linker, a 0.1M pyridine solution of 3-[(dimethylamino-methylidene)amino]-3H-1,2,4-dithiazole-3-thione (DDTT) or a 1:1 solution of 0.05M pyridine-acetonitrile was used instead of the oxidation step in Table 1 above.

After completion of oligonucleotide synthesis, a concentrated ammonia solution was added and reaction was performed at 60°C for 12 to 18 hours to cleave CPG and simultaneously remove all protective groups. Thereafter, the CPG was removed by filtration, ammonia was appropriately concentrated, and then the residue was desalted by filtration through Sephadex G-25 resin, lyophilized, and used immediately. Alternatively, the residue was purified using preparative high-performance liquid chromatography (prep-HPLC) and then precipitated with a 2-3-fold volume of cold ethanol from a 0.3M sodium chloride (NaCl) or sodium acetate (NaOAc) solution, and used.

The synthesized and purified oligonucleotides were analyzed by high-performance liquid chromatography (analytical HPLC) to confirm that the purity was 80% or more. In addition, the oligonucleotides were quantified by measuring the absorbance at a wavelength of 260 nm using an UV-VIS spectrometer, and then the molecular weights of the oligonucleotides were determined by MALDI-TOF or Q-TOF mass spectrometry before use.

### 3. Analysis of WFDC2 mRNA Expression Level in ASO-Administered Cells by Quantitative Real-Time PCR (qRT-PCR)

One day before transformation, cells that had been subcultured 5 to 10 times were cultured in a 9 cm² (6-well) culture dish (#3006, SPL) at a density of 0.25 × 10⁶ cells. The next day, after confirming that the cells reached about 80% of the area of the 9 cm² (6-well) culture dish, a transformation experiment was performed. Lipofectamine 3000 (#L3000008, Thermofisher) was used as a transformation reagent. The culture medium was replaced with Opti-MEM (#A3635101, Gipco), and cells were analyzed after treatment with 0 nM (control) to 100 nM of each ASO for 24 hours using Lipofectamine 3000, based on the protocol provided by the manufacturer.

After transformation, the medium was removed from the culture dish, and then the cells were washed twice with PBS (#ML 008-01, Welgene). Next, total RNA was extracted from the cells using TRIzol (#15596018, Ambion) according to the manufacturer's protocol. Using the extracted RNA as a template, cDNA was synthesized using the ImProm-II^{™} Reverse Transcription System (#A3800, Promega) according to the manufacturer's protocol. Thereafter, qRT-PCR was performed using the synthesized cDNA, the primers shown in Table 2 below, and TB Green^{®} Fast qPCR Mix (#RR430, Takara) according to the manufacturer's protocol. Analysis was performed using the StepOne^{™} Real-Time PCR System (#4376357, Applied Biosystems), and based on the results of qRT-PCR, the expression level of WFDC2 in the ASO-treated group was expressed as a percentage relative to 0 nM (control).

**[Table 2]**

| Primer name | Nucleotide sequence | SEQ ID NO. |
|---|---|---|
| WFDC2 1-F | TGCTCTCTGCCCAATGATAA | 3 |
| WFDC2 1-R | TTGGGAGTGACACAGGACAC | 4 |
| GAPDH-F | CTGACTTCAACAGCGACACC | 5 |
| GAPDH-R | GGTGGTCCAGGGGTCTTACT | 6 |

### 4. Analysis of WFDC2 Protein Expression Level in ASO-Administered Cells by Enzyme-Linked Immunosorbent Assay (ELISA)

One day before transformation, cells that had been subcultured 5 to 10 times were cultured in a 1.9 cm² (24-well) culture dish (#30024, SPL) at a density of 5.0 × 10⁴ cells. The next day, after confirming that the cells reached about 80% of the area of the 1.9 cm² (24-well) culture dish, a transformation experiment was performed. Lipofectamine 3000 (#L3000008, Invitrogen) was used as a transformation reagent. The culture medium was replaced with Opti-MEM (#A3635101, Gipco), and the cells were treated with 0 nM (control) to 400 nM of each ASO for 48 hours using Lipofectamine 3000, based on the protocol provided by the manufacturer. After 48 hours, the cell culture was collected in a 1.5-ml microtube (MCT-150-C, AXYGEN) and centrifuged at a speed of 1,000 rpm at 4°C for 20 minutes to settle the debris contained in the cell culture medium. Then, only the supernatant was collected in a 1.5-ml microtube and the sample was stored in a -80°C ultra-low-temperature freezer.

Using the Duoset ELISA kit for WFDC2 (DY6274-05, R&D System, Minneapolis, MN, USA), WFDC2 concentration was measured according to the following protocol provided by the manufacturer. Human WFDC2 capture antibody (#844347, R&D System) was diluted in PBS, and the dilution was dispensed into each well of a 96-well micro-plate (#DY990, R&D System), incubated at room temperature for 30 minutes, and stored in a refrigerator at 4°C for a day. Next, 100 µL of the cell culture or 100 µL of the WFDC2 recombinant protein standard solution was diluted 2-fold with reagent diluent (#DY995, R&D System), and the dilution was dispensed into each well of the 96-well micro-plate and allowed to react at room temperature for 2 hours. Then, biotinylated human WFDC2 detection antibody (#844348, R&D System) diluted in reagent diluent was dispensed into each well of the 96 well micro-plate and allowed to react at room temperature for 2 hours, and then 100 µL of streptavidin-peroxidase solution (streptavidin-HRP, #893975, R&D System) was dispensed into each well and allowed to react at room temperature for 20 minutes. Thereafter, 100 µL of tetramethylbenzidine solution (substrate solution, #DY999, R&D System) was dispensed into each well of the 96-well micro-plate and allowed to react for 7 minutes. Then, 50 µL of 2N sulfuric acid solution was dispensed into each well to inhibit the reaction, and the absorbance at a wavelength of 450 nm was measured using a microplate reader. The concentration of WFDC2 in each sample was calculated by substituting the measured absorbance of each sample into a standard curve prepared from the absorbance of a known concentration of the WFDC2 recombinant protein standard solution. The inhibition rate of WFDC2 production relative to the negative control was calculated using the following equation: Inhibition rate (%) of WFDC2 production in sample = (WFDC2 concentration of negative control) - (WFDC2 concentration of sample) / (WFDC2 concentration of negative control) × 100

### 5. Evaluation of Cancer Growth Inhibitory Effect of ASO using SNU638 Xenograft Mouse Model

SNU638 cells grown under the cell culture conditions described in the above Example were suspended in a 1:1 solution of Matrigel (#354230, Corning)/PBS (#ML 008-01, Welgene), and then injected at 3 × 10⁶ cells into each of 8-week-old male NOD.SCID mice (NOD.CB17-Prkdcsscid/NCrKoat) under inhalation anesthesia. Thereafter, the cancer cells were monitored for 3 weeks after injection for colonization and growth. 3 weeks after cancer cell transplantation, Compound 3 at concentrations of 7.5 mpk and 30 mpk was injected to each mouse twice a week for 4 weeks (a total of 8 times) via the tail vein injection route (IV group) and the subcutaneous injection route (SC group). The number (N) of mice in each of the IV 7.5 mpk group, the IV 30 mpk group, the SC 7.5 mpk group, and the SC 30 mpk group was 8, and the control group consisted of 5 mice. The cancer growth inhibitory effect of the ASO was evaluated by measuring the size (mm³) of cancer cells using vernier calipers for 28 days.

### 6. Evaluation of Cancer Growth Inhibitory Effect of ASO using SF268 Xenograft Mouse Model

SF268 cells grown under the cell culture conditions described in the above Example were suspended in a 1:1 solution of Matrigel (#354230, Corning)/PBS (#ML 008-01, Welgene), and then injected at 5 × 10⁶ cells into each of 8-week-old male NOD.SCID mice (NOD.CB17-Prkdcsscid/NCrKoat) under inhalation anesthesia. Thereafter, the cancer cells were monitored for 3 weeks after injection for colonization and growth. 3 weeks after cancer cell transplantation, Compound 3 at a concentration of 20 mpk was injected to each mouse three times a week for 4 weeks (a total of 12 times) via the tail vein injection route (IV group). The number (N) of mice in the IV 20 mpk group was 8, and the control group consisted of 4 mice. The cancer growth inhibitory effect of the ASO was evaluated by measuring the size (mm³) of cancer cells using vernier calipers for 24 days.

### 7. Statistical Analysis

All statistical analyses were performed through GraphPad prism 9.0.0, significance verification was performed using the Two-way ANOVA Multiple Comparisons test, and values indicating statistical significance are indicated by *, **, and ***. * P ≤ 0.05, ** P ≤ 0.01, and *** P ≤0.001.

### Experiment Results

### 1. Results of Preparation of ASOs

Through the antisense oligonucleotide preparation method described in the "experimental method" section, a total of 380 antisense oligonucleotides were synthesized, including a 5-8-5 or 5-10-5 MOE gapmer antisense oligonucleotide wherein the 5' and 3' wings consist of 5 continuous nucleosides modified with 2'-MOE, the gap consists of 8 to 10 continuous natural DNA nucleosides, and the internucleoside linkages are all modified with phosphorothioate, or a 3-10-3 LNA gapmer antisense oligonucleotide wherein the 5' and 3' wings consist of 3 continuous nucleosides modified with 2'-LNA, the gap consists of 10 continuous natural DNA nucleosides, and the internucleoside linkages are all modified with phosphorothioate. Table 3 below shows the nucleotide sequences including the start and stop sites of pre-mRNA (SEQ ID NO: 1) or mRNA (SEQ ID NO: 2) of WFDC2 and the gapmer motifs.

**[Table 3]**

| Compou nd No. | SEQ ID NO. | Start site of SEQ ID N: 1 | Stop site of SEQ ID N: 1 | Start site of SEQ ID N: 2 | Stop site of SEQ ID N: 2 | Sequence (5'->3') | Gapmer motif |
|---|---|---|---|---|---|---|---|
| 1 | 7 | 25 | 44 | 25 | 44 | GCGACAAGCAGGCATGGTGC | 5-10-5 MOE |
| 2 | 8 | 27 | 46 | 27 | 46 | AGGCGACAAGCAGGCATGGT | 5-10-5 MOE |
| 3 | 9 | 10285 | 10304 | 348 | 367 | CCATTGCGGCAGCATTTCAT | 5-10-5 MOE |
| 4 | 10 | 99 | 118 | N/A | N/A | CCCCACTCACCTGAGACTAG | 5-10-5 MOE |
| 5 | 11 | 194 | 213 | N/A | N/A | AATTCCCACTTCCCCAGCCT | 5-10-5 MOE |
| 6 | 12 | 196 | 215 | N/A | N/A | GGAATTCCCACTTCCCCAGC | 5-10-5 MOE |
| 7 | 13 | 258 | 277 | N/A | N/A | CCACCTCCAGCACATTGGAC | 5-10-5 MOE |
| 8 | 14 | 259 | 278 | N/A | N/A | TCCACCTCCAGCACATTGGA | 5-10-5 MOE |
| 9 | 15 | 261 | 280 | N/A | N/A | TCTCCACCTCCAGCACATTG | 5-10-5 MOE |
| 10 | 16 | 262 | 281 | N/A | N/A | CTCTCCACCTCCAGCACATT | 5-10-5 MOE |
| 11 | 17 | 268 | 287 | N/A | N/A | AGTGGTCTCTCCACCTCCAG | 5-10-5 MOE |
| 12 | 18 | 277 | 296 | N/A | N/A | GCAGCCATCAGTGGTCTCTC | 5-10-5 MOE |
| 13 | 19 | 282 | 301 | N/A | N/A | AAATTGCAGCCATCAGTGGT | 5-10-5 MOE |
| 14 | 20 | 284 | 303 | N/A | N/A | CCAAATTGCAGCCATCAGTG | 5-10-5 MOE |
| 15 | 21 | 293 | 312 | N/A | N/A | AGAATCCTCCCAAATTGCAG | 5-10-5 MOE |
| 16 | 22 | 296 | 315 | N/A | N/A | CACAGAATCCTCCCAAATTG | 5-10-5 MOE |
| 17 | 23 | 307 | 326 | N/A | N/A | TCCGCGTTCAGCACAGAATC | 5-10-5 MOE |
| 18 | 24 | 311 | 330 | N/A | N/A | AGTGTCCGCGTTCAGCACAG | 5-10-5 MOE |
| 19 | 25 | 412 | 431 | N/A | N/A | CCCTCAGATCTCAGCCCTAG | 5-10-5 MOE |
| 20 | 26 | 441 | 460 | N/A | N/A | CGAGAGCTCCCTAACCCTTG | 5-10-5 MOE |
| 21 | 27 | 442 | 461 | N/A | N/A | ACGAGAGCTCCCTAACCCTT | 5-10-5 MOE |
| 22 | 28 | 443 | 462 | N/A | N/A | TACGAGAGCTCCCTAACCCT | 5-10-5 MOE |
| 23 | 29 | 446 | 465 | N/A | N/A | GAGTACGAGAGCTCCCTAAC | 5-10-5 MOE |
| 24 | 30 | 473 | 492 | N/A | N/A | TCCAGACCAGGAGTCCCTGA | 5-10-5 MOE |
| 25 | 31 | 474 | 493 | N/A | N/A | TTCCAGACCAGGAGTCCCTG | 5-10-5 MOE |
| 26 | 32 | 478 | 497 | N/A | N/A | CTTCTTCCAGACCAGGAGTC | 5-10-5 MOE |
| 27 | 33 | 483 | 502 | N/A | N/A | GACTCCTTCTTCCAGACCAG | 5-10-5 MOE |
| 28 | 34 | 486 | 505 | N/A | N/A | AGAGACTCCTTCTTCCAGAC | 5-10-5 MOE |
| 29 | 35 | 489 | 508 | N/A | N/A | CCCAGAGACTCCTTCTTCCA | 5-10-5 MOE |
| 30 | 36 | 514 | 533 | N/A | N/A | TGGCCCTAGGAGTCCCCTTA | 5-10-5 MOE |
| 31 | 37 | 10258 | 10277 | 321 | 338 | ACACTGGCTGTCCACCTG | 5-8-5 MOE |
| 32 | 38 | 10271 | 10290 | 334 | 353 | TTTCATCTGGCCAGGACACT | 5-10-5 MOE |
| 33 | 39 | 726 | 743 | 198 | 215 | GCAGCACTTGAGGTTGTC | 5-8-5 MOE |
| 34 | 40 | 19 | 36 | 19 | 36 | CAGGCATGGTGCTATGCC | 5-8-5 MOE |
| 35 | 41 | 304 | 323 | N/A | N/A | GCGTTCAGCACAGAATCCTC | 5-10-5 MOE |
| 36 | 42 | 380 | 399 | N/A | N/A | AGCTGAGCGTCTCGGAGCTT | 5-10-5 MOE |
| 37 | 43 | 480 | 499 | N/A | N/A | TCCTTCTTCCAGACCAGGAG | 5-10-5 MOE |
| 38 | 44 | 484 | 503 | N/A | N/A | AGACTCCTTCTTCCAGACCA | 5-10-5 MOE |
| 39 | 45 | 511 | 530 | N/A | N/A | CCCTAGGAGTCCCCTTACAG | 5-10-5 MOE |
| 40 | 46 | 520 | 539 | N/A | N/A | AGTCTCTGGCCCTAGGAGTC | 5-10-5 MOE |
| 41 | 47 | 522 | 541 | N/A | N/A | TCAGTCTCTGGCCCTAGGAG | 5-10-5 MOE |
| 42 | 48 | 525 | 544 | N/A | N/A | TTCTCAGTCTCTGGCCCTAG | 5-10-5 MOE |
| 43 | 49 | 526 | 545 | N/A | N/A | ATTCTCAGTCTCTGGCCCTA | 5-10-5 MOE |
| 44 | 50 | 532 | 551 | N/A | N/A | CAAGGAATTCTCAGTCTCTG | 5-10-5 MOE |
| 45 | 51 | 534 | 553 | N/A | N/A | CCCAAGGAATTCTCAGTCTC | 5-10-5 MOE |
| 46 | 52 | 536 | 555 | N/A | N/A | ACCCCAAGGAATTCTCAGTC | 5-10-5 MOE |
| 47 | 53 | 538 | 557 | N/A | N/A | TAACCCCAAGGAATTCTCAG | 5-10-5 MOE |
| 48 | 54 | 539 | 558 | N/A | N/A | TTAACCCCAAGGAATTCTCA | 5-10-5 MOE |
| 49 | 55 | 541 | 560 | N/A | N/A | CCTTAACCCCAAGGAATTCT | 5-10-5 MOE |
| 50 | 56 | 542 | 561 | N/A | N/A | ACCTTAACCCCAAGGAATTC | 5-10-5 MOE |
| 51 | 57 | 543 | 562 | N/A | N/A | AACCTTAACCCCAAGGAATT | 5-10-5 MOE |
| 52 | 58 | 546 | 565 | N/A | N/A | CCAAACCTTAACCCCAAGGA | 5-10-5 MOE |
| 53 | 59 | 548 | 567 | N/A | N/A | CTCCAAACCTTAACCCCAAG | 5-10-5 MOE |
| 54 | 60 | 554 | 573 | N/A | N/A | CTCCTGCTCCAAACCTTAAC | 5-10-5 MOE |
| 55 | 61 | 561 | 580 | N/A | N/A | TGCCCACCTCCTGCTCCAAA | 5-10-5 MOE |
| 56 | 62 | 562 | 581 | N/A | N/A | ATGCCCACCTCCTGCTCCAA | 5-10-5 MOE |
| 57 | 63 | 10283 | 10302 | 346 | 365 | ATTGCGGCAGCATTTCATCT | 5-10-5 MOE |
| 58 | 64 | 10284 | 10303 | 347 | 366 | CATTGCGGCAGCATTTCATC | 5-10-5 MOE |
| 59 | 65 | 10286 | 10305 | 349 | 368 | GCCATTGCGGCAGCATTTCA | 5-10-5 MOE |
| 60 | 66 | 10287 | 10306 | 350 | 369 | AGCCATTGCGGCAGCATTTC | 5-10-5 MOE |
| 61 | 67 | 10288 | 10307 | 351 | 370 | CAGCCATTGCGGCAGCATTT | 5-10-5 MOE |
| 62 | 68 | 10259 | 10278 | 322 | 341 | AGGACACTGGCTGTCCACCT | 5-10-5 MOE |
| 63 | 69 | 10295 | 10314 | 358 | 377 | CTTCCCACAGCCATTGCGGC | 5-10-5 MOE |
| 64 | 70 | 10258 | 10277 | 321 | 340 | GGACACTGGCTGTCCACCTG | 5-10-5 MOE |
| 65 | 71 | 10260 | 10279 | 323 | 342 | CAGGACACTGGCTGTCCACC | 5-10-5 MOE |
| 66 | 72 | 10256 | 10275 | 319 | 338 | ACACTGGCTGTCCACCTGGC | 5-10-5 MOE |
| 67 | 73 | 10257 | 10276 | 320 | 339 | GACACTGGCTGTCCACCTGG | 5-10-5 MOE |
| 68 | 74 | 19 | 38 | 19 | 38 | AGCAGGCATGGTGCTATGCC | 5-10-5 MOE |
| 69 | 75 | 17 | 36 | 17 | 36 | CAGGCATGGTGCTATGCCCG | 5-10-5 MOE |
| 70 | 76 | N/A | N/A | 97 | 116 | TCCTGTGCCTGAGACTAGGG | 5-10-5 MOE |
| 71 | 77 | N/A | N/A | 99 | 118 | GCTCCTGTGCCTGAGACTAG | 5-10-5 MOE |
| 72 | 78 | 636 | 655 | 108 | 127 | GTCTTCTCTGCTCCTGTGCC | 5-10-5 MOE |
| 73 | 79 | 638 | 657 | 110 | 129 | CAGTCTTCTCTGCTCCTGTG | 5-10-5 MOE |
| 74 | 80 | 10299 | 10318 | 362 | 381 | ACACCTTCCCACAGCCATTG | 5-10-5 MOE |
| 75 | 81 | 10300 | 10319 | 363 | 382 | GACACCTTCCCACAGCCATT | 5-10-5 MOE |
| 76 | 82 | 11627 | 11646 | 414 | 433 | TCACTGCTCAGCCTGGTGGT | 5-10-5 MOE |
| 77 | 83 | 11633 | 11652 | 420 | 439 | CTCTCCTCACTGCTCAGCCT | 5-10-5 MOE |
| 78 | 84 | 11636 | 11655 | 423 | 442 | TTTCTCTCCTCACTGCTCAG | 5-10-5 MOE |
| 79 | 85 | 11641 | 11660 | 428 | 447 | GAAACTTTCTCTCCTCACTG | 5-10-5 MOE |
| 80 | 86 | 11645 | 11664 | 432 | 451 | GGCAGAAACTTTCTCTCCTC | 5-10-5 MOE |
| 81 | 87 | 11652 | 11671 | 439 | 458 | AGGGCCAGGCAGAAACTTTC | 5-10-5 MOE |
| 82 | 88 | 11656 | 11675 | 443 | 462 | ATGCAGGGCCAGGCAGAAAC | 5-10-5 MOE |
| 83 | 89 | 11670 | 11689 | 457 | 476 | TGGGCTGGAACCAGATGCAG | 5-10-5 MOE |
| 84 | 90 | 11704 | 11723 | 491 | 510 | GGGAATACAGAGTCCCGAAA | 5-10-5 MOE |
| 85 | 91 | 11710 | 11729 | 497 | 516 | CCAAGAGGGAATACAGAGTC | 5-10-5 MOE |
| 86 | 92 | 11723 | 11742 | 510 | 529 | AGCTGTGGTCAGCCCAAGAG | 5-10-5 MOE |
| 87 | 93 | 11748 | 11767 | 535 | 554 | TACTTTATTGGTTGGGAAAG | 5-10-5 MOE |
| 88 | 94 | 11753 | 11772 | 540 | 559 | GTGGTTACTTTATTGGTTGG | 5-10-5 MOE |
| 89 | 95 | 11758 | 11777 | 545 | 564 | TGAAAGTGGTTACTTTATTG | 5-10-5 MOE |
| 90 | 96 | 11761 | 11780 | 548 | 567 | TGCTGAAAGTGGTTACTTTA | 5-10-5 MOE |
| 91 | 97 | N/A | N/A | 94 | 113 | TGTGCCTGAGACTAGGGTGA | 5-10-5 MOE |
| 92 | 98 | 646 | 665 | 118 | 137 | GCACACGCCAGTCTTCTCTG | 5-10-5 MOE |
| 93 | 99 | 669 | 688 | 141 | 160 | TTCTGGTCAGCCTGGAGCTC | 5-10-5 MOE |
| 94 | 100 | 679 | 698 | 151 | 170 | TTGCGTGCAGTTCTGGTCAG | 5-10-5 MOE |
| 95 | 101 | 719 | 738 | 191 | 210 | ACTTGAGGTTGTCGGCGCAT | 5-10-5 MOE |
| 96 | 102 | 727 | 746 | 199 | 218 | GCTGCAGCACTTGAGGTTGT | 5-10-5 MOE |
| 97 | 103 | N/A | N/A | 236 | 255 | TATCATTGGGCAGAGAGCAG | 5-10-5 MOE |
| 98 | 104 | 10209 | 10228 | 272 | 291 | GAAAGTTAATGTTCACCTGG | 5-10-5 MOE |
| 99 | 105 | 10272 | 10291 | 335 | 354 | ATTTCATCTGGCCAGGACAC | 5-10-5 MOE |
| 100 | 106 | 10320 | 10339 | 383 | 402 | AGAAATTGGGAGTGACACAG | 5-10-5 MOE |
| 101 | 107 | N/A | N/A | 240 | 259 | TCCTTATCATTGGGCAGAGA | 5-10-5 MOE |
| 102 | 108 | N/A | N/A | 248 | 267 | AGGAACCCTCCTTATCATTG | 5-10-5 MOE |
| 103 | 109 | 10360 | 10379 | N/A | N/A | TGGCCATCAATGCACTTTCT | 5-10-5 MOE |
| 104 | 110 | 10384 | 10403 | N/A | N/A | TTTTCTCCTGTTTCCCACAA | 5-10-5 MOE |
| 105 | 111 | 10441 | 10460 | N/A | N/A | TATACCTTTCCCAACTGTCC | 5-10-5 MOE |
| 106 | 112 | 10447 | 10466 | N/A | N/A | CACTGGTATACCTTTCCCAA | 5-10-5 MOE |
| 107 | 113 | 10476 | 10495 | N/A | N/A | CTTCTTTTAGAACAGGCTGA | 5-10-5 MOE |
| 108 | 114 | 10485 | 10504 | N/A | N/A | CCTTTCACTCTTCTTTTAGA | 5-10-5 MOE |
| 109 | 115 | 10493 | 10512 | N/A | N/A | ACTACCCACCTTTCACTCTT | 5-10-5 MOE |
| 110 | 116 | 10508 | 10527 | N/A | N/A | AATGCAGCTCATCAGACTAC | 5-10-5 MOE |
| 111 | 117 | 10546 | 10565 | N/A | N/A | TTGCTTATTCTGTTCCCTCT | 5-10-5 MOE |
| 112 | 118 | 10553 | 10572 | N/A | N/A | CAAGCTCTTGCTTATTCTGT | 5-10-5 MOE |
| 113 | 119 | 10588 | 10607 | N/A | N/A | TGCTGGGATTATAGGCATGA | 5-10-5 MOE |
| 114 | 120 | 10596 | 10615 | N/A | N/A | TCCCAAAGTGCTGGGATTAT | 5-10-5 MOE |
| 115 | 121 | 10681 | 10700 | N/A | N/A | GGTATTTTTAGTAGAGACGG | 5-10-5 MOE |
| 116 | 122 | 10771 | 10790 | N/A | N/A | CAGGTTCAAGCAATTCTCCT | 5-10-5 MOE |
| 117 | 123 | 10848 | 10867 | N/A | N/A | TTGAGATGGAGTTTCGCTCT | 5-10-5 MOE |
| 118 | 124 | 10923 | 10942 | N/A | N/A | AAAGTAGCACATGACAACCA | 5-10-5 MOE |
| 119 | 125 | 10933 | 10952 | N/A | N/A | GAAATTGTTAAAAGTAGCAC | 5-10-5 MOE |
| 120 | 126 | 10944 | 10963 | N/A | N/A | TACTTTGCTGAGAAATTGTT | 5-10-5 MOE |
| 121 | 127 | 10956 | 10975 | N/A | N/A | TTATCTTCAGGTTACTTTGC | 5-10-5 MOE |
| 122 | 128 | 10966 | 10985 | N/A | N/A | ATTCTATCAGTTATCTTCAG | 5-10-5 MOE |
| 123 | 129 | 10977 | 10996 | N/A | N/A | TGCACTATTGGATTCTATCA | 5-10-5 MOE |
| 124 | 130 | 10990 | 11009 | N/A | N/A | AATTGCTCATCTCTGCACTA | 5-10-5 MOE |
| 125 | 131 | 10999 | 11018 | N/A | N/A | CTAGATTTCAATTGCTCATC | 5-10-5 MOE |
| 126 | 132 | 11008 | 11027 | N/A | N/A | ACCCCCTCTCTAGATTTCAA | 5-10-5 MOE |
| 127 | 133 | 11047 | 11066 | N/A | N/A | TTAGAGAAACCATAGTTCCC | 5-10-5 MOE |
| 128 | 134 | 11059 | 11078 | N/A | N/A | CCATAGCTTAGTTTAGAGAA | 5-10-5 MOE |
| 129 | 135 | 11108 | 11127 | N/A | N/A | CAAAGCCTATCCATGCAGTT | 5-10-5 MOE |
| 130 | 136 | 11118 | 11137 | N/A | N/A | TTCTTGACTGCAAAGCCTAT | 5-10-5 MOE |
| 131 | 137 | 11128 | 11147 | N/A | N/A | AACCAAGGTCTTCTTGACTG | 5-10-5 MOE |
| 132 | 138 | 11136 | 11155 | N/A | N/A | AACATTTGAACCAAGGTCTT | 5-10-5 MOE |
| 133 | 139 | 11146 | 11165 | N/A | N/A | TGTCAGAGCTAACATTTGAA | 5-10-5 MOE |
| 134 | 140 | 11158 | 11177 | N/A | N/A | AGGTTAGGTAAGTGTCAGAG | 5-10-5 MOE |
| 135 | 141 | 11167 | 11186 | N/A | N/A | GAGGTGAGAAGGTTAGGTAA | 5-10-5 MOE |
| 136 | 142 | 11189 | 11208 | N/A | N/A | AGCGAGATAACTGTGACTCA | 5-10-5 MOE |
| 137 | 143 | 11202 | 11221 | N/A | N/A | GCTACATTTTATAAGCGAGA | 5-10-5 MOE |
| 138 | 144 | 11231 | 11250 | N/A | N/A | GGATTATCTGATCAATTAGA | 5-10-5 MOE |
| 139 | 145 | 11238 | 11257 | N/A | N/A | TAAAATGGGATTATCTGATC | 5-10-5 MOE |
| 140 | 146 | 11248 | 11267 | N/A | N/A | TTCTTTAGGTTAAAATGGGA | 5-10-5 MOE |
| 141 | 147 | 11258 | 11277 | N/A | N/A | CCATGCCTTCTTCTTTAGGT | 5-10-5 MOE |
| 142 | 148 | 11273 | 11292 | N/A | N/A | TATTAGGAAGTTCTGCCATG | 5-10-5 MOE |
| 143 | 149 | 11281 | 11300 | N/A | N/A | CCTTCTACTATTAGGAAGTT | 5-10-5 MOE |
| 144 | 150 | 11288 | 11307 | N/A | N/A | CAAGAATCCTTCTACTATTA | 5-10-5 MOE |
| 145 | 151 | 11294 | 11313 | N/A | N/A | TCTCCCCAAGAATCCTTCTA | 5-10-5 MOE |
| 146 | 152 | 11430 | 11449 | N/A | N/A | AGACATACTCTTCTCCTTCA | 5-10-5 MOE |
| 147 | 153 | 11439 | 11458 | N/A | N/A | ACAGGTTCTAGACATACTCT | 5-10-5 MOE |
| 148 | 154 | 11450 | 11469 | N/A | N/A | TTGCATTTTCTACAGGTTCT | 5-10-5 MOE |
| 149 | 155 | 11458 | 11477 | N/A | N/A | GGCTCTGCTTGCATTTTCTA | 5-10-5 MOE |
| 150 | 156 | 11533 | 11552 | N/A | N/A | CAGCTTAAATCCTAGACCAG | 5-10-5 MOE |
| 151 | 157 | 11540 | 11559 | N/A | N/A | GTACCTGCAGCTTAAATCCT | 5-10-5 MOE |
| 152 | 158 | 11561 | 11580 | N/A | N/A | TATCCATTAGACTAGGCAGG | 5-10-5 MOE |
| 153 | 159 | 11571 | 11590 | N/A | N/A | TCAACAACAATATCCATTAG | 5-10-5 MOE |
| 154 | 160 | 11581 | 11600 | N/A | N/A | AACAATACCATCAACAACAA | 5-10-5 MOE |
| 155 | 161 | 906 | 925 | N/A | N/A | ACTGACGGATCTGGTTTCAA | 5-10-5 MOE |
| 156 | 162 | 1004 | 1023 | N/A | N/A | ATGCACCAGAGACTCGAATC | 5-10-5 MOE |
| 157 | 163 | 1246 | 1265 | N/A | N/A | TGTAAAGTTCCTTTCCGCCT | 5-10-5 MOE |
| 158 | 164 | 1283 | 1302 | N/A | N/A | ACACATCTTTAAGATGAGCG | 5-10-5 MOE |
| 159 | 165 | 1325 | 1344 | N/A | N/A | TGACCTTGGGCAAGAACATT | 5-10-5 MOE |
| 160 | 166 | 1388 | 1407 | N/A | N/A | AGTTGGCCTGAAAGGCAAAA | 5-10-5 MOE |
| 161 | 167 | 1407 | 1426 | N/A | N/A | TTCTTCGAATCACTCAGCCA | 5-10-5 MOE |
| 162 | 168 | 1416 | 1435 | N/A | N/A | TTCCTCACTTTCTTCGAATC | 5-10-5 MOE |
| 163 | 169 | 1424 | 1443 | N/A | N/A | AGGGAGGATTCCTCACTTTC | 5-10-5 MOE |
| 164 | 170 | 1460 | 1479 | N/A | N/A | ATTGACTGAAAGACGACGAA | 5-10-5 MOE |
| 165 | 171 | 1472 | 1491 | N/A | N/A | AGAGTGGAAGAGATTGACTG | 5-10-5 MOE |
| 166 | 172 | 1481 | 1500 | N/A | N/A | TCAATCCTTAGAGTGGAAGA | 5-10-5 MOE |
| 167 | 173 | 1488 | 1507 | N/A | N/A | CGCTCACTCAATCCTTAGAG | 5-10-5 MOE |
| 168 | 174 | 1520 | 1539 | N/A | N/A | CAGCAAGCACCTTTGAGAGA | 5-10-5 MOE |
| 169 | 175 | 1551 | 1570 | N/A | N/A | TGTTCTGATAGCCTGGTGGA | 5-10-5 MOE |
| 170 | 176 | 1567 | 1586 | N/A | N/A | TGTTTAAGCCACCCCCTGTT | 5-10-5 MOE |
| 171 | 177 | 1575 | 1594 | N/A | N/A | TTCCATGCTGTTTAAGCCAC | 5-10-5 MOE |
| 172 | 178 | 1580 | 1599 | N/A | N/A | TGAGATTCCATGCTGTTTAA | 5-10-5 MOE |
| 173 | 179 | 1593 | 1612 | N/A | N/A | GAGAACGGAAAGCTGAGATT | 5-10-5 MOE |
| 174 | 180 | 1601 | 1620 | N/A | N/A | TTCCTCTGGAGAACGGAAAG | 5-10-5 MOE |
| 175 | 181 | 1619 | 1638 | N/A | N/A | CCTCTAATCCTCAGCATTTT | 5-10-5 MOE |
| 176 | 182 | 1630 | 1649 | N/A | N/A | TCCTATCCCTGCCTCTAATC | 5-10-5 MOE |
| 177 | 183 | 1645 | 1664 | N/A | N/A | TTGGTTCTCTCCAGGTCCTA | 5-10-5 MOE |
| 178 | 184 | 1657 | 1676 | N/A | N/A | CATTACAGTACCTTGGTTCT | 5-10-5 MOE |
| 179 | 185 | 1664 | 1683 | N/A | N/A | AGAACATCATTACAGTACCT | 5-10-5 MOE |
| 180 | 186 | 1674 | 1693 | N/A | N/A | TTCTGGATAAAGAACATCAT | 5-10-5 MOE |
| 181 | 187 | 1681 | 1700 | N/A | N/A | GGCTGTATTCTGGATAAAGA | 5-10-5 MOE |
| 182 | 188 | 1724 | 1743 | N/A | N/A | TGCTGGCATCTTCAAGGAAA | 5-10-5 MOE |
| 183 | 189 | 1745 | 1764 | N/A | N/A | ATTACAAACCTTCCCAGCCT | 5-10-5 MOE |
| 184 | 190 | 1757 | 1776 | N/A | N/A | TTGGGTCACCGTATTACAAA | 5-10-5 MOE |
| 185 | 191 | 1762 | 1781 | N/A | N/A | GTGTCTTGGGTCACCGTATT | 5-10-5 MOE |
| 186 | 192 | 1884 | 1903 | N/A | N/A | TCCAAATCTTCCTCTCTCCT | 5-10-5 MOE |
| 187 | 193 | 1945 | 1964 | N/A | N/A | AAGGAGGGAGTCTTTGCTGT | 5-10-5 MOE |
| 188 | 194 | 1967 | 1986 | N/A | N/A | GAACAAATTCTTCATCCATC | 5-10-5 MOE |
| 189 | 195 | 1974 | 1993 | N/A | N/A | GATAAGGGAACAAATTCTTC | 5-10-5 MOE |
| 190 | 196 | 1988 | 2007 | N/A | N/A | GTCTGTCCATCTGAGATAAG | 5-10-5 MOE |
| 191 | 197 | 2100 | 2119 | N/A | N/A | AAACAACATGCTCAGGATCA | 5-10-5 MOE |
| 192 | 198 | 2125 | 2144 | N/A | N/A | TCTTAGAGCAGAGGCTCAGA | 5-10-5 MOE |
| 193 | 199 | 2134 | 2153 | N/A | N/A | TTATTCCCATCTTAGAGCAG | 5-10-5 MOE |
| 194 | 200 | 2160 | 2179 | N/A | N/A | TCATGCCAATCCTATAGAGT | 5-10-5 MOE |
| 195 | 201 | 2171 | 2190 | N/A | N/A | TCATTTAGTCCTCATGCCAA | 5-10-5 MOE |
| 196 | 202 | 2194 | 2213 | N/A | N/A | ATAGACACTTTAGATGCATT | 5-10-5 MOE |
| 197 | 203 | 2205 | 2224 | N/A | N/A | TCTTCTCACTAATAGACACT | 5-10-5 MOE |
| 198 | 204 | 2215 | 2234 | N/A | N/A | GTATGGAGTATCTTCTCACT | 5-10-5 MOE |
| 199 | 205 | 2222 | 2241 | N/A | N/A | CCACTGTGTATGGAGTATCT | 5-10-5 MOE |
| 200 | 206 | 2230 | 2249 | N/A | N/A | AAAACAAGCCACTGTGTATG | 5-10-5 MOE |
| 201 | 207 | 2238 | 2257 | N/A | N/A | TTATGATCAAAACAAGCCAC | 5-10-5 MOE |
| 202 | 208 | 2247 | 2266 | N/A | N/A | GGGAACCATTTATGATCAAA | 5-10-5 MOE |
| 203 | 209 | 2258 | 2277 | N/A | N/A | GGAAATAAATAGGGAACCAT | 5-10-5 MOE |
| 204 | 210 | 2269 | 2288 | N/A | N/A | TGGGCTGCCCTGGAAATAAA | 5-10-5 MOE |
| 205 | 211 | 2275 | 2294 | N/A | N/A | TAATATTGGGCTGCCCTGGA | 5-10-5 MOE |
| 206 | 212 | 2285 | 2304 | N/A | N/A | TAGGAACCTGTAATATTGGG | 5-10-5 MOE |
| 207 | 213 | 2293 | 2312 | N/A | N/A | GAAAGGAGTAGGAACCTGTA | 5-10-5 MOE |
| 208 | 214 | 2304 | 2323 | N/A | N/A | TTCATTCTCTTGAAAGGAGT | 5-10-5 MOE |
| 209 | 215 | 2325 | 2344 | N/A | N/A | TGACTTCCTAATACAAAGAA | 5-10-5 MOE |
| 210 | 216 | 2334 | 2353 | N/A | N/A | CAACAGTCTTGACTTCCTAA | 5-10-5 MOE |
| 211 | 217 | 2341 | 2360 | N/A | N/A | AAGTTGCCAACAGTCTTGAC | 5-10-5 MOE |
| 212 | 218 | 892 | 911 | N/A | N/A | TTTCAACCGCCTTGACTTTC | 5-10-5 MOE |
| 213 | 219 | 899 | 918 | N/A | N/A | GATCTGGTTTCAACCGCCTT | 5-10-5 MOE |
| 214 | 220 | 950 | 969 | N/A | N/A | TTCAACGACGCCTTTGTCTA | 5-10-5 MOE |
| 215 | 221 | 997 | 1016 | N/A | N/A | AGAGACTCGAATCCCAGCTA | 5-10-5 MOE |
| 216 | 222 | 1263 | 1282 | N/A | N/A | GGGCATTTTCCTAAATCTGT | 5-10-5 MOE |
| 217 | 223 | 1294 | 1313 | N/A | N/A | GATGCTCCCTTACACATCTT | 5-10-5 MOE |
| 218 | 224 | 1302 | 1321 | N/A | N/A | TTCTCACCGATGCTCCCTTA | 5-10-5 MOE |
| 219 | 225 | 1366 | 1385 | N/A | N/A | TGAACCCTCACTATGACTCC | 5-10-5 MOE |
| 220 | 226 | 2061 | 2080 | N/A | N/A | AACTAGGATTCAAACCTCCA | 5-10-5 MOE |
| 221 | 227 | 2154 | 2173 | N/A | N/A | CAATCCTATAGAGTTTGGAA | 5-10-5 MOE |
| 222 | 228 | 9179 | 9198 | N/A | N/A | GAACTGGGTGATTAGCTGTA | 5-10-5 MOE |
| 223 | 229 | 9282 | 9301 | N/A | N/A | CATTCATCAACTGAGGTACA | 5-10-5 MOE |
| 224 | 230 | 9325 | 9344 | N/A | N/A | CACTTCCCAGGTTAGATAGA | 5-10-5 MOE |
| 225 | 231 | 9339 | 9358 | N/A | N/A | ACACAATCACACTCCACTTC | 5-10-5 MOE |
| 226 | 232 | 9365 | 9384 | N/A | N/A | ACACGTCCTTCCACTAACAA | 5-10-5 MOE |
| 227 | 233 | 9388 | 9407 | N/A | N/A | ATTCATCCATGCAGAGAGAA | 5-10-5 MOE |
| 228 | 234 | 9426 | 9445 | N/A | N/A | TTGCAGTCTCTCAGCACCAT | 5-10-5 MOE |
| 229 | 235 | 9459 | 9478 | N/A | N/A | AATCTTCACTCATACCCACA | 5-10-5 MOE |
| 230 | 236 | 9468 | 9487 | N/A | N/A | CACACTTATAATCTTCACTC | 5-10-5 MOE |
| 231 | 237 | 9506 | 9525 | N/A | N/A | ACTTAAAGTGTAAAGTACAG | 5-10-5 MOE |
| 232 | 238 | 9513 | 9532 | N/A | N/A | TGCTGCCACTTAAAGTGTAA | 5-10-5 MOE |
| 233 | 239 | 9522 | 9541 | N/A | N/A | TAAGCACTATGCTGCCACTT | 5-10-5 MOE |
| 234 | 240 | 9532 | 9551 | N/A | N/A | CTGCCATGGTTAAGCACTAT | 5-10-5 MOE |
| 235 | 241 | 9696 | 9715 | N/A | N/A | CAATAATCCTAAAAGAAGGG | 5-10-5 MOE |
| 236 | 242 | 9715 | 9734 | N/A | N/A | TTAACTCTTCTAATTCTCAC | 5-10-5 MOE |
| 237 | 243 | 9733 | 9752 | N/A | N/A | TCAAGCCCCTAACATATATT | 5-10-5 MOE |
| 238 | 244 | 9746 | 9765 | N/A | N/A | TACATGCCAGGTATCAAGCC | 5-10-5 MOE |
| 239 | 245 | 9767 | 9786 | N/A | N/A | GAACACTTAATTAGCTCTTA | 5-10-5 MOE |
| 240 | 246 | 9797 | 9816 | N/A | N/A | TACTCTGTAGTTATGAGAAA | 5-10-5 MOE |
| 241 | 247 | 9802 | 9821 | N/A | N/A | CAAACTACTCTGTAGTTATG | 5-10-5 MOE |
| 242 | 248 | 9807 | 9826 | N/A | N/A | AATATCAAACTACTCTGTAG | 5-10-5 MOE |
| 243 | 249 | 9814 | 9833 | N/A | N/A | TCCCTGAAATATCAAACTAC | 5-10-5 MOE |
| 244 | 250 | 9843 | 9862 | N/A | N/A | AGCAGTGGGAAACAAACATA | 5-10-5 MOE |
| 245 | 251 | 9865 | 9884 | N/A | N/A | TACAACATAGGGTTGTTGTG | 5-10-5 MOE |
| 246 | 252 | 9874 | 9893 | N/A | N/A | AGTGCTAATTACAACATAGG | 5-10-5 MOE |
| 247 | 253 | 9885 | 9904 | N/A | N/A | GCCAGTGGAACAGTGCTAAT | 5-10-5 MOE |
| 248 | 254 | 9895 | 9914 | N/A | N/A | GTCTTTAGGTGCCAGTGGAA | 5-10-5 MOE |
| 249 | 255 | 10120 | 10139 | N/A | N/A | TTAACTTGTACCTGCAGCAT | 5-10-5 MOE |
| 250 | 256 | 10124 | 10143 | N/A | N/A | GAGATTAACTTGTACCTGCA | 5-10-5 MOE |
| 251 | 257 | 10287 | 10302 | 350 | 365 | ATTGCGGCAGCATTTC | 3-10-3 LNA |
| 252 | 258 | 11706 | 11721 | 493 | 508 | GAATACAGAGTCCCGA | 3-10-3 LNA |
| 253 | 259 | 11754 | 11769 | 541 | 556 | GTTACTTTATTGGTTG | 3-10-3 LNA |
| 254 | 260 | 2350 | 2369 | N/A | N/A | AATGTTGTTAAGTTGCCAAC | 5-10-5 MOE |
| 255 | 261 | 2732 | 2751 | N/A | N/A | AAGGAAATGTGGCACGTCTA | 5-10-5 MOE |
| 256 | 262 | 2830 | 2849 | N/A | N/A | ATGCCCAAGACACATGCACA | 5-10-5 MOE |
| 257 | 263 | 2838 | 2857 | N/A | N/A | CAACGCAAATGCCCAAGACA | 5-10-5 MOE |
| 258 | 264 | 3117 | 3136 | N/A | N/A | GTTAGAATGGAGATCCTTAA | 5-10-5 MOE |
| 259 | 265 | 3256 | 3275 | N/A | N/A | TAGAACGTGGGCGAAGGATA | 5-10-5 MOE |
| 260 | 266 | 3421 | 3440 | N/A | N/A | GATCCAATTAAACTACAGAG | 5-10-5 MOE |
| 261 | 267 | 3854 | 3873 | N/A | N/A | GGAAGCATCACGCTAACTGA | 5-10-5 MOE |
| 262 | 268 | 4286 | 4305 | N/A | N/A | TAAGAAACTGCAGTAAAGTC | 5-10-5 MOE |
| 263 | 269 | 4294 | 4313 | N/A | N/A | TAAGCTGATAAGAAACTGCA | 5-10-5 MOE |
| 264 | 270 | 4999 | 5018 | N/A | N/A | ACAAAGAGGAGTTGCTGGTA | 5-10-5 MOE |
| 265 | 271 | 5814 | 5833 | N/A | N/A | AACACTAATTAGCAAATGCA | 5-10-5 MOE |
| 266 | 272 | 5960 | 5979 | N/A | N/A | AAAGATATCCAAGACTTGAA | 5-10-5 MOE |
| 267 | 273 | 6015 | 6034 | N/A | N/A | GAAACTTTAACCCCCACTGT | 5-10-5 MOE |
| 268 | 274 | 6038 | 6057 | N/A | N/A | AGACTCCGACATAATAATAG | 5-10-5 MOE |
| 269 | 275 | 6548 | 6567 | N/A | N/A | ACAACTGGAATCAGCAACTG | 5-10-5 MOE |
| 270 | 276 | 6553 | 6572 | N/A | N/A | AAGGAACAACTGGAATCAGC | 5-10-5 MOE |
| 271 | 277 | 6610 | 6629 | N/A | N/A | TTTTCTCATCAACAGGCCTG | 5-10-5 MOE |
| 272 | 278 | 6785 | 6804 | N/A | N/A | CCCTTCAGACTAACTGCAGA | 5-10-5 MOE |
| 273 | 279 | 6811 | 6830 | N/A | N/A | AAGGTTGGGTTACCCACTAT | 5-10-5 MOE |
| 274 | 280 | 7221 | 7240 | N/A | N/A | TGAAGCCTATACAAGTATCA | 5-10-5 MOE |
| 275 | 281 | 7231 | 7250 | N/A | N/A | GAGAACGTCATGAAGCCTAT | 5-10-5 MOE |
| 276 | 282 | 7334 | 7353 | N/A | N/A | AAGAAAGGTAAGAACCTTGA | 5-10-5 MOE |
| 277 | 283 | 7347 | 7366 | N/A | N/A | CTAACCCCAATGCAAGAAAG | 5-10-5 MOE |
| 278 | 284 | 7355 | 7374 | N/A | N/A | AGCATGTTCTAACCCCAATG | 5-10-5 MOE |
| 279 | 285 | 7880 | 7899 | N/A | N/A | ATTTCCGTGTCTCCTGACTG | 5-10-5 MOE |
| 280 | 286 | 7888 | 7907 | N/A | N/A | AGTCCCTGATTTCCGTGTCT | 5-10-5 MOE |
| 281 | 287 | 8257 | 8276 | N/A | N/A | AACTCTCAAGCTTGGTAGGG | 5-10-5 MOE |
| 282 | 288 | 8268 | 8287 | N/A | N/A | AGTTGACCTGGAACTCTCAA | 5-10-5 MOE |
| 283 | 289 | 8590 | 8609 | N/A | N/A | TCTGGAGCAGATACTACACT | 5-10-5 MOE |
| 284 | 290 | 8600 | 8619 | N/A | N/A | AATAGTGCACTCTGGAGCAG | 5-10-5 MOE |
| 285 | 291 | 8614 | 8633 | N/A | N/A | ACTGTGCCGTGAGGAATAGT | 5-10-5 MOE |
| 286 | 292 | 8770 | 8789 | N/A | N/A | ATTTTCAAATGAGGTACGTG | 5-10-5 MOE |
| 287 | 293 | 8807 | 8826 | N/A | N/A | TCCCAGTGAATCAATGCAGA | 5-10-5 MOE |
| 288 | 294 | 8860 | 8879 | N/A | N/A | GATTTAGGTTCAGCTTTCAA | 5-10-5 MOE |
| 289 | 295 | 9153 | 9172 | N/A | N/A | AAGGAAAGTGGCTGACAGAT | 5-10-5 MOE |
| 290 | 296 | 9171 | 9190 | N/A | N/A | TGATTAGCTGTAGAGGACAA | 5-10-5 MOE |
| 291 | 297 | 9975 | 9994 | N/A | N/A | CAAATGGAATCACAGGACCT | 5-10-5 MOE |
| 292 | 298 | 9983 | 10002 | N/A | N/A | CCTGCTCCCAAATGGAATCA | 5-10-5 MOE |
| 293 | 299 | 10006 | 10025 | N/A | N/A | AAGATCACCTGCAAATCCCT | 5-10-5 MOE |
| 294 | 300 | 10079 | 10098 | N/A | N/A | TGGGCTTCACATACAGCAGA | 5-10-5 MOE |
| 295 | 301 | 10132 | 10151 | N/A | N/A | GATACAGGGAGATTAACTTG | 5-10-5 MOE |
| 296 | 302 | 10155 | 10174 | N/A | N/A | AGTAAGGGTAAGTGGGCAGA | 5-10-5 MOE |
| 297 | 303 | 10355 | 10374 | N/A | N/A | ATCAATGCACTTTCTCTTTC | 5-10-5 MOE |
| 298 | 304 | 10357 | 10376 | N/A | N/A | CCATCAATGCACTTTCTCTT | 5-10-5 MOE |
| 299 | 305 | 10362 | 10381 | N/A | N/A | CCTGGCCATCAATGCACTTT | 5-10-5 MOE |
| 300 | 306 | 10470 | 10489 | N/A | N/A | TTAGAACAGGCTGAGGGTCA | 5-10-5 MOE |
| 301 | 307 | 10474 | 10493 | N/A | N/A | TCTTTTAGAACAGGCTGAGG | 5-10-5 MOE |
| 302 | 308 | 10478 | 10497 | N/A | N/A | CTCTTCTTTTAGAACAGGCT | 5-10-5 MOE |
| 303 | 309 | 10581 | 10600 | N/A | N/A | ATTATAGGCATGAGCCACCA | 5-10-5 MOE |
| 304 | 310 | 10649 | 10668 | N/A | N/A | TTGGTCAGGCTGGTCTTGAA | 5-10-5 MOE |
| 305 | 311 | 10662 | 10681 | N/A | N/A | GGGTTTCTCCATGTTGGTCA | 5-10-5 MOE |
| 306 | 312 | 10715 | 10734 | N/A | N/A | CCACCACACCCATTAAATTT | 5-10-5 MOE |
| 307 | 313 | 10764 | 10783 | N/A | N/A | AAGCAATTCTCCTGCCTCAG | 5-10-5 MOE |
| 308 | 314 | 10778 | 10797 | N/A | N/A | TGTCTCCCAGGTTCAAGCAA | 5-10-5 MOE |
| 309 | 315 | 10799 | 10818 | N/A | N/A | ATCTCAGCTCACCACAACCT | 5-10-5 MOE |
| 310 | 316 | 10813 | 10832 | N/A | N/A | AGTGCAATGGCGTGATCTCA | 5-10-5 MOE |
| 311 | 317 | 10839 | 10858 | N/A | N/A | AGTTTCGCTCTTGTTGCCCA | 5-10-5 MOE |
| 312 | 318 | 10858 | 10877 | N/A | N/A | TGTTTTTAATTTGAGATGGA | 5-10-5 MOE |
| 313 | 319 | 10881 | 10900 | N/A | N/A | CACCAAGCTGTTTTTTGTTT | 5-10-5 MOE |
| 314 | 320 | 10886 | 10905 | N/A | N/A | GGTACCACCAAGCTGTTTTT | 5-10-5 MOE |
| 315 | 321 | 10899 | 10918 | N/A | N/A | ATTTCAACCCAAGGGTACCA | 5-10-5 MOE |
| 316 | 322 | 10907 | 10926 | N/A | N/A | ACCACGAGATTTCAACCCAA | 5-10-5 MOE |
| 317 | 323 | 10915 | 10934 | N/A | N/A | ACATGACAACCACGAGATTT | 5-10-5 MOE |
| 318 | 324 | 10975 | 10994 | N/A | N/A | CACTATTGGATTCTATCAGT | 5-10-5 MOE |
| 319 | 325 | 10979 | 10998 | N/A | N/A | TCTGCACTATTGGATTCTAT | 5-10-5 MOE |
| 320 | 326 | 10997 | 11016 | N/A | N/A | AGATTTCAATTGCTCATCTC | 5-10-5 MOE |
| 321 | 327 | 11000 | 11019 | N/A | N/A | TCTAGATTTCAATTGCTCAT | 5-10-5 MOE |
| 322 | 328 | 11185 | 11204 | N/A | N/A | AGATAACTGTGACTCAGGGA | 5-10-5 MOE |
| 323 | 329 | 11191 | 11210 | N/A | N/A | TAAGCGAGATAACTGTGACT | 5-10-5 MOE |
| 324 | 330 | 11448 | 11467 | N/A | N/A | GCATTTTCTACAGGTTCTAG | 5-10-5 MOE |
| 325 | 331 | 11452 | 11471 | N/A | N/A | GCTTGCATTTTCTACAGGTT | 5-10-5 MOE |
| 326 | 332 | 11455 | 11474 | N/A | N/A | TCTGCTTGCATTTTCTACAG | 5-10-5 MOE |
| 327 | 333 | 11538 | 11557 | N/A | N/A | ACCTGCAGCTTAAATCCTAG | 5-10-5 MOE |
| 328 | 334 | 11542 | 11561 | N/A | N/A | GAGTACCTGCAGCTTAAATC | 5-10-5 MOE |
| 329 | 335 | 11544 | 11563 | N/A | N/A | AGGAGTACCTGCAGCTTAAA | 5-10-5 MOE |
| 330 | 336 | 4580 | 4599 | N/A | N/A | GAAGGTATCTCAAAGTAACA | 5-10-5 MOE |
| 331 | 337 | 4942 | 4961 | N/A | N/A | AGAGCCTCCTCCCTAATTCA | 5-10-5 MOE |
| 332 | 338 | 5362 | 5381 | N/A | N/A | CACAAGCTGGGTTTTTGAAA | 5-10-5 MOE |
| 333 | 339 | 5714 | 5733 | N/A | N/A | GCATAACCTTACATGGAAAC | 5-10-5 MOE |
| 334 | 340 | 5722 | 5741 | N/A | N/A | TCAAGACCGCATAACCTTAC | 5-10-5 MOE |
| 335 | 341 | 9193 | 9212 | N/A | N/A | AAAACACAGGCACAGAACTG | 5-10-5 MOE |
| 336 | 342 | 9201 | 9220 | N/A | N/A | TTGTGTGTAAAACACAGGCA | 5-10-5 MOE |
| 337 | 343 | 9262 | 9281 | N/A | N/A | TCATGGTTGCTTCCAACTTT | 5-10-5 MOE |
| 338 | 344 | 9290 | 9309 | N/A | N/A | GCCAAATCCATTCATCAACT | 5-10-5 MOE |
| 339 | 345 | 9384 | 9403 | N/A | N/A | AGAGAAACCACACGTCCTTC | 5-10-5 MOE |
| 340 | 346 | 9409 | 9428 | N/A | N/A | CATGACCCTCCAAATACACT | 5-10-5 MOE |
| 341 | 347 | 9558 | 9577 | N/A | N/A | CTGCAGAGTCTATGTGTTAA | 5-10-5 MOE |
| 342 | 348 | 9565 | 9584 | N/A | N/A | AATCTAGCTGCAGAGTCTAT | 5-10-5 MOE |
| 343 | 349 | 9578 | 9597 | N/A | N/A | TTGAACCCCAAGAAATCTAG | 5-10-5 MOE |
| 344 | 350 | 9851 | 9870 | N/A | N/A | GTTGTGAAAGCAGTGGGAAA | 5-10-5 MOE |
| 345 | 351 | 10322 | 10341 | 385 | 404 | TCAGAAATTGGGAGTGACAC | 5-10-5 MOE |
| 346 | 352 | N/A | N/A | 396 | 415 | GTGGCTGGAGCTCAGAAATT | 5-10-5 MOE |
| 347 | 353 | 11639 | 11658 | 426 | 445 | AACTTTCTCTCCTCACTGCT | 5-10-5 MOE |
| 348 | 354 | 1382 | 1401 | N/A | N/A | CCTGAAAGGCAAAAGCTGAA | 5-10-5 MOE |
| 349 | 355 | 4864 | 4883 | N/A | N/A | AATTTCAGGTTAATATCCCT | 5-10-5 MOE |
| 350 | 356 | 5327 | 5346 | N/A | N/A | GTCTGACTGCTAGCCATACT | 5-10-5 MOE |
| 351 | 357 | 5342 | 5361 | N/A | N/A | AACATCAACAAAATAGTCTG | 5-10-5 MOE |
| 352 | 358 | 5370 | 5389 | N/A | N/A | AATGAATTCACAAGCTGGGT | 5-10-5 MOE |
| 353 | 359 | 5423 | 5442 | N/A | N/A | GATCAGAGAGAACTAAAGGA | 5-10-5 MOE |
| 354 | 360 | 5728 | 5747 | N/A | N/A | ACTCACTCAAGACCGCATAA | 5-10-5 MOE |
| 355 | 361 | 7586 | 7605 | N/A | N/A | AAGGCCCATCCAAAGATCAT | 5-10-5 MOE |
| 356 | 362 | 7895 | 7914 | N/A | N/A | TCAAGTGAGTCCCTGATTTC | 5-10-5 MOE |
| 357 | 363 | 8542 | 8561 | N/A | N/A | CAAGAGATTCCTTTGGGTGC | 5-10-5 MOE |
| 358 | 364 | 8694 | 8713 | N/A | N/A | AGTAGGGTAGGGCATCATCT | 5-10-5 MOE |
| 359 | 365 | 8764 | 8783 | N/A | N/A | AAATGAGGTACGTGGCTCAT | 5-10-5 MOE |
| 360 | 366 | 8855 | 8874 | N/A | N/A | AGGTTCAGCTTTCAAGATGG | 5-10-5 MOE |
| 361 | 367 | 9163 | 9182 | N/A | N/A | TGTAGAGGACAAGGAAAGTG | 5-10-5 MOE |
| 362 | 368 | 9270 | 9289 | N/A | N/A | GAGGTACATCATGGTTGCTT | 5-10-5 MOE |
| 363 | 369 | 9374 | 9393 | N/A | N/A | AGAGAAACCACACGTCCTTC | 5-10-5 MOE |
| 364 | 370 | 9401 | 9420 | N/A | N/A | TCCAAATACACTCATTCATC | 5-10-5 MOE |
| 365 | 371 | 9595 | 9614 | N/A | N/A | CAGAATTGAGGCAGAATTTG | 5-10-5 MOE |
| 366 | 372 | 9604 | 9623 | N/A | N/A | AAGGTCACACAGAATTGAGG | 5-10-5 MOE |
| 367 | 373 | 9669 | 9688 | N/A | N/A | CTGTTAATATTTCCTTGTGT | 5-10-5 MOE |
| 368 | 374 | 9676 | 9695 | N/A | N/A | GAGGGATCTGTTAATATTTC | 5-10-5 MOE |
| 369 | 375 | 10318 | 10337 | 381 | 400 | AAATTGGGAGTGACACAGGA | 5-10-5 MOE |
| 370 | 376 | 11642 | 11661 | 429 | 448 | AGAAACTTTCTCTCCTCACT | 5-10-5 MOE |
| 371 | 377 | 11644 | 11663 | 431 | 450 | GCAGAAACTTTCTCTCCTCA | 5-10-5 MOE |
| 372 | 378 | 11700 | 11719 | 487 | 506 | ATACAGAGTCCCGAAAAAGG | 5-10-5 MOE |
| 373 | 379 | 11703 | 11722 | 490 | 509 | GGAATACAGAGTCCCGAAAA | 5-10-5 MOE |
| 374 | 380 | 11751 | 11770 | 538 | 557 | GGTTACTTTATTGGTTGGGA | 5-10-5 MOE |
| 375 | 381 | 11752 | 11771 | 539 | 558 | TGGTTACTTTATTGGTTGGG | 5-10-5 MOE |
| 376 | 382 | 11754 | 11773 | 541 | 560 | AGTGGTTACTTTATTGGTTG | 5-10-5 MOE |
| 377 | 383 | 286 | 305 | N/A | N/A | TCCCAAATTGCAGCCATCAG | 5-10-5 MOE |
| 378 | 384 | 301 | 320 | N/A | N/A | TTCAGCACAGAATCCTCCCA | 5-10-5 MOE |
| 379 | 385 | 527 | 546 | N/A | N/A | AATTCTCAGTCTCTGGCCCT | 5-10-5 MOE |
| 380 | 386 | 531 | 550 | N/A | N/A | AAGGAATTCTCAGTCTCTGG | 5-10-5 MOE |

### 2. Evaluation of Effects of ASOs on mRNA Level

It was examined whether the ASOs prepared above reduced the expression of WFDC2 mRNA in the human glioblastoma cell line SF268. Table 4 shows the results of expressing the WFDC2 expression level upon 100 nM ASO treatment as a percentage relative to 0 nM (control).

**[Table 4]**

| Compound No. | SEQ ID NO. | Inhibition % (100 nM) |
|---|---|---|
| 1 | 7 | 78 |
| 2 | 8 | 80 |
| 3 | 9 | 92 |
| 14 | 20 | 45 |
| 32 | 38 | 80 |
| 33 | 39 | 71 |
| 36 | 42 | 39 |
| 37 | 43 | 42 |
| 40 | 46 | 46 |
| 41 | 47 | 47 |
| 42 | 48 | 39 |
| 43 | 49 | 65 |
| 57 | 63 | 57 |
| 58 | 64 | 71 |
| 59 | 65 | 87 |
| 60 | 66 | 72 |
| 62 | 68 | 68 |
| 63 | 69 | 62 |
| 67 | 73 | 63 |
| 70 | 76 | 59 |
| 71 | 77 | 77 |
| 72 | 78 | 65 |
| 74 | 80 | 58 |
| 76 | 82 | 74 |
| 77 | 83 | 69 |
| 79 | 85 | 66 |
| 80 | 86 | 86 |
| 81 | 87 | 59 |
| 82 | 88 | 61 |
| 83 | 89 | 81 |
| 88 | 94 | 52 |
| 92 | 98 | 54 |
| 103 | 109 | 78 |
| 106 | 112 | 55 |
| 107 | 113 | 75 |
| 108 | 114 | 59 |
| 113 | 119 | 78 |
| 114 | 120 | 87 |
| 115 | 121 | 72 |
| 116 | 122 | 76 |
| 117 | 123 | 72 |
| 118 | 124 | 62 |
| 123 | 129 | 69 |
| 125 | 131 | 77 |
| 129 | 135 | 61 |
| 130 | 136 | 65 |
| 134 | 140 | 46 |
| 135 | 141 | 41 |
| 136 | 142 | 80 |
| 140 | 146 | 23 |
| 142 | 148 | 73 |
| 148 | 154 | 78 |
| 149 | 155 | 79 |
| 150 | 156 | 65 |
| 151 | 157 | 77 |
| 152 | 158 | 61 |
| 155 | 161 | 64 |
| 156 | 162 | 62 |
| 157 | 163 | 64 |
| 158 | 164 | 68 |
| 159 | 165 | 80 |
| 160 | 166 | 69 |
| 161 | 167 | 52 |
| 162 | 168 | 48 |
| 163 | 169 | 80 |
| 170 | 176 | 68 |
| 171 | 177 | 62 |
| 172 | 178 | 54 |
| 173 | 179 | 48 |
| 174 | 180 | 48 |
| 175 | 181 | 52 |
| 176 | 182 | 50 |
| 178 | 184 | 55 |
| 179 | 185 | 57 |
| 185 | 191 | 65 |
| 199 | 205 | 74 |
| 202 | 208 | 66 |
| 203 | 209 | 68 |
| 204 | 210 | 79 |
| 205 | 211 | 59 |
| 206 | 212 | 61 |
| 207 | 213 | 68 |
| 208 | 214 | 77 |
| 209 | 215 | 85 |
| 212 | 218 | 69 |
| 213 | 219 | 56 |
| 214 | 220 | 67 |
| 215 | 221 | 51 |
| 216 | 222 | 59 |
| 217 | 223 | 44 |
| 219 | 225 | 65 |
| 220 | 226 | 68 |
| 221 | 227 | 61 |
| 222 | 228 | 67 |
| 223 | 229 | 81 |
| 224 | 230 | 42 |
| 225 | 231 | 48 |
| 226 | 232 | 51 |
| 227 | 233 | 50 |
| 228 | 234 | 36 |
| 229 | 235 | 31 |
| 230 | 236 | 62 |
| 231 | 237 | 75 |
| 232 | 238 | 82 |
| 233 | 239 | 87 |
| 234 | 240 | 83 |
| 237 | 243 | 80 |
| 238 | 244 | 77 |
| 239 | 245 | 80 |
| 240 | 246 | 65 |
| 241 | 247 | 81 |
| 242 | 248 | 79 |
| 243 | 249 | 65 |
| 244 | 250 | 85 |
| 245 | 251 | 82 |
| 246 | 252 | 88 |
| 247 | 253 | 81 |
| 248 | 254 | 76 |
| 249 | 255 | 86 |
| 250 | 256 | 73 |
| 251 | 257 | 55 |
| 252 | 258 | 53 |
| 253 | 259 | 59 |
| 254 | 260 | 51 |
| 258 | 264 | 62 |
| 275 | 281 | 58 |
| 276 | 282 | 62 |
| 277 | 283 | 58 |
| 278 | 284 | 75 |
| 279 | 285 | 77 |
| 280 | 286 | 71 |
| 281 | 287 | 66 |
| 282 | 288 | 45 |
| 283 | 289 | 78 |
| 284 | 290 | 76 |
| 285 | 291 | 83 |
| 286 | 292 | 80 |
| 287 | 293 | 75 |
| 289 | 295 | 69 |
| 290 | 296 | 60 |
| 291 | 297 | 52 |
| 292 | 298 | 63 |
| 293 | 299 | 54 |
| 294 | 300 | 73 |
| 304 | 310 | 83 |
| 307 | 313 | 85 |
| 308 | 314 | 86 |
| 310 | 316 | 85 |
| 311 | 317 | 77 |
| 314 | 320 | 70 |
| 321 | 327 | 66 |
| 324 | 330 | 67 |
| 325 | 331 | 56 |
| 327 | 333 | 55 |
| 329 | 335 | 68 |
| 330 | 336 | 72 |
| 332 | 338 | 62 |
| 333 | 339 | 53 |
| 334 | 340 | 58 |
| 337 | 343 | 70 |
| 343 | 349 | 36 |
| 365 | 371 | 62 |
| 366 | 372 | 45 |
| 367 | 373 | 59 |
| 368 | 374 | 55 |
| 369 | 375 | 60 |
| 370 | 376 | 84 |
| 371 | 377 | 82 |
| 372 | 378 | 79 |
| 373 | 379 | 89 |
| 374 | 380 | 96 |
| 375 | 381 | 92 |
| 376 | 382 | 90 |
| 377 | 383 | 82 |
| 380 | 386 | 65 |

In addition, Tables 5 and 6 below show compounds that showed a concentration-dependent inhibitory effect on mRNA production in the SNU638 and SF268 cell lines.

**[Table 5]**

| Compound No. | SEQ ID NO. | Inhibition % (1 nM) | Inhibition % (3 nM) | Inhibition % (10 nM) | Inhibition % (30 nM) |
|---|---|---|---|---|---|
| 1 | 7 | -6 | -3 | 20 | 50 |
| 2 | 8 | -2 | 0 | 11 | 34 |
| 3 | 9 | 4 | 31 | 75 | 93 |

**[Table 6]**

| Compound No. | SEQ ID NO. | Inhibition % (1 nM) | Inhibition % (3 nM) | Inhibition % (10 nM) |
|---|---|---|---|---|
| 3 | 9 | 17.3 | 52.4 | 81 |
| 58 | 64 | 26.6 | 38.1 | 74.3 |
| 59 | 65 | 39.7 | 51.1 | 74.8 |
| 63 | 69 | 10.7 | 49.9 | 53.1 |

### 3. Evaluation of Effects of ASOs on Protein Level

It was examined by ELISA whether the prepared ASOs reduced the expression of WFDC2 protein in the human gastric cancer cell line SNU638 and the pancreatic cancer cell line PANC1.

As a result of examining the degree of ASO-induced decrease in WFDC2 protein expression in the gastric cancer cell line SNU638 and the pancreatic cancer cell line PANC1 by ELISA, as shown in Tables 7 and 8 below, it was confirmed that the following compounds significantly reduced WFDC2 protein expression in each of the SNU638 cell line and the PANC1 cell line: Compound 2, Compound 3, Compound 32, Compound 43, Compound 59, Compound 80, Compound 103, Compound 113, Compound 114, Compound 115, Compound 116, Compound 117, Compound 125, Compound 130, Compound 136, Compound 142, Compound 148, Compound 149, Compound 151, Compound 159, Compound 163, Compound 199, Compound 204, Compound 208, Compound 209, Compound 223, Compound 232, Compound 233, Compound 234, Compound 237, Compound 239, Compound 244, Compound 245, Compound 246, Compound 247, Compound 248, Compound 249, Compound 279, Compound 285, Compound 286, Compound 294, Compound 304, Compound 307, Compound 308, Compound 310, Compound 311, Compound 314, Compound 324, Compound 325, Compound 330, Compound 337, Compound 370, Compound 373, Compound 374, Compound 375, Compound 376, and Compound 377.

**[Table 7]**

| Compound No. | SEQ ID NO. | Inhibition % (100 nM) |
|---|---|---|
| 1 | 7 | 67 |
| 2 | 8 | 74 |
| 3 | 9 | 62 |
| 4 | 10 | 0 |
| 5 | 11 | 0 |
| 6 | 12 | 13 |
| 7 | 13 | 0 |
| 8 | 14 | 0 |
| 9 | 15 | 0 |
| 10 | 16 | 27 |
| 11 | 17 | 9 |
| 12 | 18 | 0 |
| 13 | 19 | 0 |
| 14 | 20 | 25 |
| 15 | 21 | 0 |
| 16 | 22 | 0 |
| 17 | 23 | 21 |
| 18 | 24 | 32 |
| 19 | 25 | 12 |
| 20 | 26 | 20 |
| 21 | 27 | 0 |
| 22 | 28 | 0 |
| 23 | 29 | 36 |
| 24 | 30 | 30 |
| 25 | 31 | 31 |
| 26 | 32 | 0 |
| 27 | 33 | 7 |
| 28 | 34 | 0 |
| 29 | 35 | 0 |
| 30 | 36 | 23 |
| 31 | 37 | 13 |
| 32 | 38 | 73 |
| 33 | 39 | 74 |
| 34 | 40 | 45 |
| 35 | 41 | 25 |
| 36 | 42 | 18 |
| 37 | 43 | 24 |
| 38 | 44 | 0 |
| 39 | 45 | 24 |
| 40 | 46 | 35 |
| 41 | 47 | 41 |
| 42 | 48 | 30 |
| 43 | 49 | 55 |
| 44 | 50 | 25 |
| 45 | 51 | 11 |
| 46 | 52 | 3 |
| 47 | 53 | 21 |
| 48 | 54 | 22 |
| 49 | 55 | 4 |
| 50 | 56 | 17 |
| 51 | 57 | 0 |
| 52 | 58 | 0 |
| 53 | 59 | 6 |
| 54 | 60 | 9 |
| 55 | 61 | 7 |
| 56 | 62 | 0 |
| 57 | 63 | 53 |
| 58 | 64 | 51 |
| 59 | 65 | 59 |
| 60 | 66 | 57 |
| 61 | 67 | 30 |
| 62 | 68 | 57 |
| 63 | 69 | 53 |
| 64 | 70 | 40 |
| 65 | 71 | 38 |
| 66 | 72 | 35 |
| 67 | 73 | 55 |
| 68 | 74 | 47 |
| 69 | 75 | 41 |
| 70 | 76 | 46 |
| 71 | 77 | 59 |
| 72 | 78 | 55 |
| 73 | 79 | 47 |
| 74 | 80 | 53 |
| 75 | 81 | 38 |
| 76 | 82 | 56 |
| 77 | 83 | 71 |
| 78 | 84 | 48 |
| 79 | 85 | 57 |
| 80 | 86 | 78 |
| 81 | 87 | 61 |
| 82 | 88 | 56 |
| 83 | 89 | 75 |
| 84 | 90 | 42 |
| 85 | 91 | 31 |
| 86 | 92 | 31 |
| 87 | 93 | 36 |
| 88 | 94 | 48 |
| 89 | 95 | 31 |
| 90 | 96 | 22 |
| 91 | 97 | 34 |
| 92 | 98 | 44 |
| 93 | 99 | 28 |
| 94 | 100 | 30 |
| 95 | 101 | 36 |
| 96 | 102 | 29 |
| 97 | 103 | 25 |
| 98 | 104 | 14 |
| 99 | 105 | 25 |
| 100 | 106 | 36 |
| 101 | 107 | 19 |
| 102 | 108 | 18 |
| 103 | 109 | 67 |
| 104 | 110 | 0 |
| 105 | 111 | 0 |
| 106 | 112 | 40 |
| 107 | 113 | 61 |
| 108 | 114 | 41 |
| 109 | 115 | 2 |
| 110 | 116 | 0 |
| 111 | 117 | 33 |
| 112 | 118 | 8 |
| 113 | 119 | 91 |
| 114 | 120 | 94 |
| 115 | 121 | 88 |
| 116 | 122 | 93 |
| 117 | 123 | 94 |
| 118 | 124 | 49 |
| 119 | 125 | 13 |
| 120 | 126 | 43 |
| 121 | 127 | 30 |
| 122 | 128 | 12 |
| 123 | 129 | 65 |
| 124 | 130 | 37 |
| 125 | 131 | 73 |
| 126 | 132 | 0 |
| 127 | 133 | 0 |
| 128 | 134 | 37 |
| 129 | 135 | 57 |
| 130 | 136 | 52 |
| 131 | 137 | 32 |
| 132 | 138 | 15 |
| 133 | 139 | 5 |
| 134 | 140 | 30 |
| 135 | 141 | 30 |
| 136 | 142 | 77 |
| 137 | 143 | 26 |
| 138 | 144 | 0 |
| 139 | 145 | 0 |
| 140 | 146 | 42 |
| 141 | 147 | 29 |
| 142 | 148 | 67 |
| 143 | 149 | 24 |
| 144 | 150 | 0 |
| 145 | 151 | 0 |
| 146 | 152 | 0 |
| 147 | 153 | 0 |
| 148 | 154 | 65 |
| 149 | 155 | 58 |
| 150 | 156 | 42 |
| 151 | 157 | 62 |
| 152 | 158 | 41 |
| 153 | 159 | 8 |
| 154 | 160 | 33 |
| 155 | 161 | 44 |
| 156 | 162 | 44 |
| 157 | 163 | 45 |
| 158 | 164 | 48 |
| 159 | 165 | 55 |
| 160 | 166 | 47 |
| 161 | 167 | 42 |
| 162 | 168 | 44 |
| 163 | 169 | 53 |
| 164 | 170 | 33 |
| 165 | 171 | 35 |
| 166 | 172 | 44 |
| 167 | 173 | 45 |
| 168 | 174 | 40 |
| 169 | 175 | 43 |
| 170 | 176 | 53 |
| 171 | 177 | 48 |
| 172 | 178 | 41 |
| 173 | 179 | 41 |
| 174 | 180 | 48 |
| 175 | 181 | 48 |
| 176 | 182 | 45 |
| 177 | 183 | 36 |
| 178 | 184 | 48 |
| 179 | 185 | 48 |
| 180 | 186 | 24 |
| 181 | 187 | 35 |
| 182 | 188 | 45 |
| 183 | 189 | 28 |
| 184 | 190 | 38 |
| 185 | 191 | 52 |
| 186 | 192 | 21 |
| 187 | 193 | 11 |
| 188 | 194 | 29 |
| 189 | 195 | 23 |
| 190 | 196 | 40 |
| 191 | 197 | 31 |
| 192 | 198 | 18 |
| 193 | 199 | 35 |
| 194 | 200 | 36 |
| 195 | 201 | 27 |
| 196 | 202 | 35 |
| 197 | 203 | 29 |
| 198 | 204 | 9 |
| 199 | 205 | 76 |
| 200 | 206 | 18 |
| 201 | 207 | 18 |
| 202 | 208 | 57 |
| 203 | 209 | 57 |
| 204 | 210 | 77 |
| 205 | 211 | 47 |
| 206 | 212 | 50 |
| 207 | 213 | 55 |
| 208 | 214 | 58 |
| 209 | 215 | 57 |
| 210 | 216 | 38 |
| 211 | 217 | 40 |
| 212 | 218 | 53 |
| 213 | 219 | 45 |
| 214 | 220 | 54 |
| 215 | 221 | 42 |
| 216 | 222 | 53 |
| 217 | 223 | 51 |
| 218 | 224 | 39 |
| 219 | 225 | 46 |
| 220 | 226 | 48 |
| 221 | 227 | 43 |
| 222 | 228 | 47 |
| 223 | 229 | 57 |
| 224 | 230 | 45 |
| 225 | 231 | 44 |
| 226 | 232 | 46 |
| 227 | 233 | 45 |
| 228 | 234 | 40 |
| 229 | 235 | 33 |
| 230 | 236 | 51 |
| 231 | 237 | 61 |
| 232 | 238 | 65 |
| 233 | 239 | 67 |
| 234 | 240 | 68 |
| 235 | 241 | 45 |
| 236 | 242 | 42 |
| 237 | 243 | 62 |
| 238 | 244 | 63 |
| 239 | 245 | 63 |
| 240 | 246 | 54 |
| 241 | 247 | 62 |
| 242 | 248 | 62 |
| 243 | 249 | 58 |
| 244 | 250 | 69 |
| 245 | 251 | 71 |
| 246 | 252 | 73 |
| 247 | 253 | 74 |
| 248 | 254 | 73 |
| 249 | 255 | 71 |
| 250 | 256 | 65 |
| 251 | 257 | 47 |
| 252 | 258 | 53 |
| 253 | 259 | 51 |
| 254 | 260 | 46 |
| 255 | 261 | 43 |
| 256 | 262 | 37 |
| 257 | 263 | 33 |
| 258 | 264 | 49 |
| 259 | 265 | 14 |
| 260 | 266 | 11 |
| 261 | 267 | 40 |
| 262 | 268 | 31 |
| 263 | 269 | 10 |
| 264 | 270 | 34 |
| 265 | 271 | 29 |
| 266 | 272 | 20 |
| 267 | 273 | 28 |
| 268 | 274 | 6 |
| 269 | 275 | 20 |
| 270 | 276 | 9 |
| 271 | 277 | 40 |
| 272 | 278 | 19 |
| 273 | 279 | 27 |
| 274 | 280 | 41 |
| 275 | 281 | 49 |
| 276 | 282 | 52 |
| 277 | 283 | 50 |
| 278 | 284 | 56 |
| 279 | 285 | 64 |
| 280 | 286 | 56 |
| 281 | 287 | 57 |
| 282 | 288 | 49 |
| 283 | 289 | 59 |
| 284 | 290 | 58 |
| 285 | 291 | 69 |
| 286 | 292 | 63 |
| 287 | 293 | 62 |
| 288 | 294 | 32 |
| 289 | 295 | 51 |
| 290 | 296 | 47 |
| 291 | 297 | 41 |
| 292 | 298 | 46 |
| 293 | 299 | 42 |
| 294 | 300 | 67 |
| 295 | 301 | 29 |
| 296 | 302 | 19 |
| 297 | 303 | 0 |
| 298 | 304 | 22 |
| 299 | 305 | 52 |
| 300 | 306 | 30 |
| 301 | 307 | 45 |
| 302 | 308 | 54 |
| 303 | 309 | 44 |
| 304 | 310 | 74 |
| 305 | 311 | 66 |
| 306 | 312 | 61 |
| 307 | 313 | 77 |
| 308 | 314 | 79 |
| 309 | 315 | 46 |
| 310 | 316 | 83 |
| 311 | 317 | 72 |
| 312 | 318 | 37 |
| 313 | 319 | 38 |
| 314 | 320 | 72 |
| 315 | 321 | 59 |
| 316 | 322 | 46 |
| 317 | 323 | 57 |
| 318 | 324 | 0 |
| 319 | 325 | 57 |
| 320 | 326 | 41 |
| 321 | 327 | 43 |
| 322 | 328 | 67 |
| 323 | 329 | 59 |
| 324 | 330 | 72 |
| 325 | 331 | 34 |
| 326 | 332 | 43 |
| 327 | 333 | 62 |
| 328 | 334 | 28 |
| 329 | 335 | 52 |
| 330 | 336 | 57 |
| 331 | 337 | 38 |
| 332 | 338 | 49 |
| 333 | 339 | 44 |
| 334 | 340 | 48 |
| 335 | 341 | 43 |
| 336 | 342 | 40 |
| 337 | 343 | 55 |
| 338 | 344 | 24 |
| 339 | 345 | 24 |
| 340 | 346 | 39 |
| 341 | 347 | 36 |
| 342 | 348 | 38 |
| 343 | 349 | 48 |
| 344 | 350 | 37 |
| 345 | 351 | 31 |
| 346 | 352 | 27 |
| 347 | 353 | 44 |
| 348 | 354 | 27 |
| 349 | 355 | 32 |
| 350 | 356 | 29 |
| 351 | 357 | 0 |
| 352 | 358 | 4 |
| 353 | 359 | 24 |
| 354 | 360 | 18 |
| 355 | 361 | 26 |
| 356 | 362 | 23 |
| 357 | 363 | 41 |
| 358 | 364 | 14 |
| 359 | 365 | 0 |
| 360 | 366 | 0 |
| 361 | 367 | 0 |
| 362 | 368 | 0 |
| 363 | 369 | 17 |
| 364 | 370 | 2 |
| 365 | 371 | 51 |
| 366 | 372 | 50 |
| 367 | 373 | 50 |
| 368 | 374 | 48 |
| 369 | 375 | 52 |
| 370 | 376 | 61 |
| 371 | 377 | 60 |
| 372 | 378 | 54 |
| 373 | 379 | 67 |
| 374 | 380 | 81 |
| 375 | 381 | 80 |
| 376 | 382 | 80 |
| 377 | 383 | 72 |
| 378 | 384 | 41 |
| 379 | 385 | 37 |
| 380 | 386 | 46 |

**[Table 8]**

| Compound No. | SEQ ID NO. | Inhibition % (100 nM) |
|---|---|---|
| 1 | 7 | 86 |
| 2 | 8 | 61 |
| 3 | 9 | 52 |
| 4 | 10 | 0 |
| 5 | 11 | 1 |
| 6 | 12 | 50 |
| 7 | 13 | 29 |
| 8 | 14 | 30 |
| 9 | 15 | 18 |
| 10 | 16 | 36 |
| 11 | 17 | 0 |
| 12 | 18 | 0 |
| 13 | 19 | 0 |
| 14 | 20 | 78 |
| 15 | 21 | 0 |
| 16 | 22 | 0 |
| 17 | 23 | 21 |
| 18 | 24 | 42 |
| 19 | 25 | 0 |
| 20 | 26 | 46 |
| 21 | 27 | 0 |
| 22 | 28 | 26 |
| 23 | 29 | 61 |
| 24 | 30 | 74 |
| 25 | 31 | 43 |
| 26 | 32 | 42 |
| 27 | 33 | 14 |
| 28 | 34 | 0 |
| 29 | 35 | 0 |
| 30 | 36 | 84 |
| 31 | 37 | 57 |
| 32 | 38 | 84 |
| 33 | 39 | 53 |
| 34 | 40 | 62 |
| 35 | 41 | 79 |
| 36 | 42 | 91 |
| 37 | 43 | 88 |
| 38 | 44 | 0 |
| 39 | 45 | 76 |
| 40 | 46 | 98 |
| 41 | 47 | 99 |
| 42 | 48 | 93 |
| 43 | 49 | 89 |
| 44 | 50 | 42 |
| 45 | 51 | 37 |
| 46 | 52 | 40 |
| 47 | 53 | 0 |
| 48 | 54 | 0 |
| 49 | 55 | 45 |
| 50 | 56 | 24 |
| 51 | 57 | 0 |
| 52 | 58 | 0 |
| 53 | 59 | 0 |
| 54 | 60 | 0 |
| 55 | 61 | 35 |
| 56 | 62 | 22 |
| 57 | 63 | 0 |
| 58 | 64 | 12 |
| 59 | 65 | 64 |
| 60 | 66 | 23 |
| 61 | 67 | 0 |
| 62 | 68 | 0 |
| 63 | 69 | 15 |
| 64 | 70 | 7 |
| 65 | 71 | 5 |
| 66 | 72 | 0 |
| 67 | 73 | 12 |
| 68 | 74 | 21 |
| 69 | 75 | 15 |
| 70 | 76 | 17 |
| 71 | 77 | 25 |
| 72 | 78 | 20 |
| 73 | 79 | 10 |
| 74 | 80 | 22 |
| 75 | 81 | 8 |
| 76 | 82 | 30 |
| 77 | 83 | 64 |
| 78 | 84 | 27 |
| 79 | 85 | 35 |
| 80 | 86 | 85 |
| 81 | 87 | 31 |
| 82 | 88 | 32 |
| 83 | 89 | 55 |
| 84 | 90 | 26 |
| 85 | 91 | 71 |
| 86 | 92 | 0 |
| 87 | 93 | 9 |
| 88 | 94 | 11 |
| 89 | 95 | 0 |
| 90 | 96 | 0 |
| 91 | 97 | 7 |
| 92 | 98 | 15 |
| 93 | 99 | 0 |
| 94 | 100 | 62 |
| 95 | 101 | 55 |
| 96 | 102 | 41 |
| 97 | 103 | 44 |
| 98 | 104 | 32 |
| 99 | 105 | 27 |
| 100 | 106 | 12 |
| 101 | 107 | 39 |
| 102 | 108 | 0 |
| 103 | 109 | 68 |
| 104 | 110 | 43 |
| 105 | 111 | 32 |
| 106 | 112 | 62 |
| 107 | 113 | 56 |
| 108 | 114 | 83 |
| 109 | 115 | 34 |
| 110 | 116 | 21 |
| 111 | 117 | 33 |
| 112 | 118 | 0 |
| 113 | 119 | 97 |
| 114 | 120 | 99 |
| 115 | 121 | 97 |
| 116 | 122 | 97 |
| 117 | 123 | 98 |
| 118 | 124 | 59 |
| 119 | 125 | 0 |
| 120 | 126 | 0 |
| 121 | 127 | 77 |
| 122 | 128 | 18 |
| 123 | 129 | 62 |
| 124 | 130 | 18 |
| 125 | 131 | 79 |
| 126 | 132 | 30 |
| 127 | 133 | 28 |
| 128 | 134 | 22 |
| 129 | 135 | 72 |
| 130 | 136 | 85 |
| 131 | 137 | 70 |
| 132 | 138 | 78 |
| 133 | 139 | 65 |
| 134 | 140 | 82 |
| 135 | 141 | 80 |
| 136 | 142 | 91 |
| 137 | 143 | 72 |
| 138 | 144 | 60 |
| 139 | 145 | 27 |
| 140 | 146 | 72 |
| 141 | 147 | 49 |
| 142 | 148 | 92 |
| 143 | 149 | 63 |
| 144 | 150 | 53 |
| 145 | 151 | 65 |
| 146 | 152 | 29 |
| 147 | 153 | 31 |
| 148 | 154 | 91 |
| 149 | 155 | 97 |
| 150 | 156 | 83 |
| 151 | 157 | 91 |
| 152 | 158 | 62 |
| 153 | 159 | 44 |
| 154 | 160 | 58 |
| 155 | 161 | 67 |
| 156 | 162 | 56 |
| 157 | 163 | 69 |
| 158 | 164 | 71 |
| 159 | 165 | 88 |
| 160 | 166 | 65 |
| 161 | 167 | 54 |
| 162 | 168 | 62 |
| 163 | 169 | 82 |
| 164 | 170 | 50 |
| 165 | 171 | 56 |
| 166 | 172 | 60 |
| 167 | 173 | 58 |
| 168 | 174 | 50 |
| 169 | 175 | 51 |
| 170 | 176 | 78 |
| 171 | 177 | 50 |
| 172 | 178 | 49 |
| 173 | 179 | 35 |
| 174 | 180 | 21 |
| 175 | 181 | 33 |
| 176 | 182 | 21 |
| 177 | 183 | 10 |
| 178 | 184 | 15 |
| 179 | 185 | 31 |
| 180 | 186 | 0 |
| 181 | 187 | 12 |
| 182 | 188 | 29 |
| 183 | 189 | 35 |
| 184 | 190 | 24 |
| 185 | 191 | 69 |
| 186 | 192 | 0 |
| 187 | 193 | 5 |
| 188 | 194 | 15 |
| 189 | 195 | 0 |
| 190 | 196 | 22 |
| 191 | 197 | 40 |
| 192 | 198 | 25 |
| 193 | 199 | 33 |
| 194 | 200 | 21 |
| 195 | 201 | 16 |
| 196 | 202 | 17 |
| 197 | 203 | 14 |
| 198 | 204 | 0 |
| 199 | 205 | 95 |
| 200 | 206 | 75 |
| 201 | 207 | 63 |
| 202 | 208 | 44 |
| 203 | 209 | 47 |
| 204 | 210 | 92 |
| 205 | 211 | 65 |
| 206 | 212 | 69 |
| 207 | 213 | 71 |
| 208 | 214 | 79 |
| 209 | 215 | 88 |
| 210 | 216 | 66 |
| 211 | 217 | 51 |
| 212 | 218 | 69 |
| 213 | 219 | 65 |
| 214 | 220 | 47 |
| 215 | 221 | 38 |
| 216 | 222 | 63 |
| 217 | 223 | 52 |
| 218 | 224 | 44 |
| 219 | 225 | 61 |
| 220 | 226 | 58 |
| 221 | 227 | 76 |
| 222 | 228 | 78 |
| 223 | 229 | 84 |
| 224 | 230 | 60 |
| 225 | 231 | 62 |
| 226 | 232 | 58 |
| 227 | 233 | 49 |
| 228 | 234 | 45 |
| 229 | 235 | 28 |
| 230 | 236 | 57 |
| 231 | 237 | 69 |
| 232 | 238 | 72 |
| 233 | 239 | 82 |
| 234 | 240 | 78 |
| 235 | 241 | 55 |
| 236 | 242 | 50 |
| 237 | 243 | 77 |
| 238 | 244 | 61 |
| 239 | 245 | 72 |
| 240 | 246 | 38 |
| 241 | 247 | 70 |
| 242 | 248 | 72 |
| 243 | 249 | 70 |
| 244 | 250 | 85 |
| 245 | 251 | 93 |
| 246 | 252 | 98 |
| 247 | 253 | 95 |
| 248 | 254 | 92 |
| 249 | 255 | 95 |
| 250 | 256 | 72 |
| 251 | 257 | 63 |
| 252 | 258 | 66 |
| 253 | 259 | 67 |
| 254 | 260 | 22 |
| 255 | 261 | 31 |
| 256 | 262 | 40 |
| 257 | 263 | 28 |
| 258 | 264 | 56 |
| 259 | 265 | 0 |
| 260 | 266 | 10 |
| 261 | 267 | 27 |
| 262 | 268 | 25 |
| 263 | 269 | 0 |
| 264 | 270 | 12 |
| 265 | 271 | 9 |
| 266 | 272 | 5 |
| 267 | 273 | 17 |
| 268 | 274 | 0 |
| 269 | 275 | 21 |
| 270 | 276 | 0 |
| 271 | 277 | 15 |
| 272 | 278 | 0 |
| 273 | 279 | 16 |
| 274 | 280 | 39 |
| 275 | 281 | 59 |
| 276 | 282 | 69 |
| 277 | 283 | 63 |
| 278 | 284 | 71 |
| 279 | 285 | 78 |
| 280 | 286 | 65 |
| 281 | 287 | 30 |
| 282 | 288 | 61 |
| 283 | 289 | 75 |
| 284 | 290 | 67 |
| 285 | 291 | 83 |
| 286 | 292 | 79 |
| 287 | 293 | 70 |
| 288 | 294 | 0 |
| 289 | 295 | 69 |
| 290 | 296 | 26 |
| 291 | 297 | 19 |
| 292 | 298 | 39 |
| 293 | 299 | 25 |
| 294 | 300 | 79 |
| 295 | 301 | 0 |
| 296 | 302 | 0 |
| 297 | 303 | 0 |
| 298 | 304 | 18 |
| 299 | 305 | 64 |
| 300 | 306 | 32 |
| 301 | 307 | 61 |
| 302 | 308 | 59 |
| 303 | 309 | 77 |
| 304 | 310 | 82 |
| 305 | 311 | 43 |
| 306 | 312 | 75 |
| 307 | 313 | 87 |
| 308 | 314 | 94 |
| 309 | 315 | 87 |
| 310 | 316 | 89 |
| 311 | 317 | 82 |
| 312 | 318 | 29 |
| 313 | 319 | 58 |
| 314 | 320 | 69 |
| 315 | 321 | 74 |
| 316 | 322 | 65 |
| 317 | 323 | 55 |
| 318 | 324 | 54 |
| 319 | 325 | 51 |
| 320 | 326 | 57 |
| 321 | 327 | 86 |
| 322 | 328 | 79 |
| 323 | 329 | 46 |
| 324 | 330 | 88 |
| 325 | 331 | 95 |
| 326 | 332 | 51 |
| 327 | 333 | 86 |
| 328 | 334 | 74 |
| 329 | 335 | 88 |
| 330 | 336 | 68 |
| 331 | 337 | 65 |
| 332 | 338 | 71 |
| 333 | 339 | 61 |
| 334 | 340 | 66 |
| 335 | 341 | 52 |
| 336 | 342 | 58 |
| 337 | 343 | 79 |
| 338 | 344 | 42 |
| 339 | 345 | 39 |
| 340 | 346 | 51 |
| 341 | 347 | 50 |
| 342 | 348 | 49 |
| 343 | 349 | 55 |
| 344 | 350 | 43 |
| 345 | 351 | 25 |
| 346 | 352 | 19 |
| 347 | 353 | 33 |
| 348 | 354 | 15 |
| 349 | 355 | 18 |
| 350 | 356 | 18 |
| 351 | 357 | 0 |
| 352 | 358 | 0 |
| 353 | 359 | 12 |
| 354 | 360 | 6 |
| 355 | 361 | 13 |
| 356 | 362 | 18 |
| 357 | 363 | 22 |
| 358 | 364 | 10 |
| 359 | 365 | 0 |
| 360 | 366 | 0 |
| 361 | 367 | 0 |
| 362 | 368 | 0 |
| 363 | 369 | 0 |
| 364 | 370 | 0 |
| 365 | 371 | 33 |
| 366 | 372 | 65 |
| 367 | 373 | 67 |
| 368 | 374 | 44 |
| 369 | 375 | 56 |
| 370 | 376 | 79 |
| 371 | 377 | 82 |
| 372 | 378 | 75 |
| 373 | 379 | 84 |
| 374 | 380 | 95 |
| 375 | 381 | 92 |
| 376 | 382 | 88 |
| 377 | 383 | 77 |
| 378 | 384 | 50 |
| 379 | 385 | 42 |
| 380 | 386 | 59 |

In addition, as shown in Tables 9 and 10 below, it was confirmed that Compound 1, Compound 3, Compound 57, Compound 58, Compound 76, Compound 77, Compound 79, Compound 80, Compound 88, Compound 113, Compound 114, Compound 115, Compound 116, Compound 117, Compound 125, and Compound 136 reduced WFDC2 expression in the SNU638 cell line in a concentration-dependent manner, and Compound 2, Compound 14, Compound 24, Compound 30, Compound 32, Compound 36, Compound 37, Compound 40, Compound 41, Compound 42, and Compound 43 reduced WFDC2 expression in the PANC1 cell line in a concentration-dependent manner.

**[Table 9]**

| Compound No. | SEQ ID NO. | Inhibition % (12.5 nM) | Inhibition % (25 nM) | Inhibition % (50 nM) | Inhibition % (100 nM) | Inhibition % (200 nM) | Inhibition % (400 nM) |
|---|---|---|---|---|---|---|---|
| 1 | 7 | 72 | 81 | 81 | 77 | 66 | - |
| 3 | 9 | 51 | 53 | 52 | 53 | 53 | - |
| 57 | 63 | - | 45 | 50 | 62 | 67 | 68 |
| 58 | 64 | - | 54 | 67 | 69 | 72 | 70 |
| 76 | 82 | - | 34 | 57 | 63 | 63 | 65 |
| 77 | 83 | - | 28 | 53 | 56 | 60 | 63 |
| 79 | 85 | - | 54 | 44 | 48 | 63 | 64 |
| 80 | 86 | - | 46 | 57 | 59 | 62 | 69 |
| 88 | 93 | - | 42 | 48 | 51 | 55 | 47 |
| 113 | 119 | 72 | 74 | 84 | 70 | 87 | - |
| 114 | 120 | 78 | 77 | 74 | 83 | 79 | - |
| 115 | 121 | 81 | 80 | 83 | 81 | 72 | - |
| 116 | 122 | 91 | 89 | 89 | 96 | 89 | - |
| 117 | 123 | 58 | 81 | 77 | 83 | 62 | - |
| 125 | 131 | 48 | 55 | 50 | 82 | 65 | - |
| 136 | 142 | 44 | 63 | 73 | 75 | 73 | - |

**[Table 10]**

| Compound No. | SEQ ID NO. | Inhibition % (25 nM) | Inhibition % (50 nM) | Inhibition % (100 nM) | Inhibition % (200 nM) | Inhibition % (400 nM) |
|---|---|---|---|---|---|---|
| 2 | 8 | 71 | 33 | 86 | 68 | 79 |
| 14 | 20 | 20 | 50 | 69 | 57 | 76 |
| 24 | 30 | 83 | 93 | 94 | 96 | 98 |
| 30 | 36 | 61 | 96 | 97 | 98 | 99 |
| 32 | 38 | 9 | 62 | 83 | 94 | 80 |
| 36 | 42 | 29 | 36 | 77 | 95 | 95 |
| 37 | 43 | 55 | 73 | 85 | 90 | 93 |
| 40 | 46 | 74 | 95 | 95 | 98 | 97 |
| 41 | 47 | 73 | 87 | 91 | 95 | 94 |
| 42 | 48 | 53 | 69 | 96 | 100 | 100 |
| 43 | 49 | 32 | 64 | 57 | 49 | 100 |

### 4. Cancer Growth Inhibitory Effect of Inhibition of WFDC2 Expression

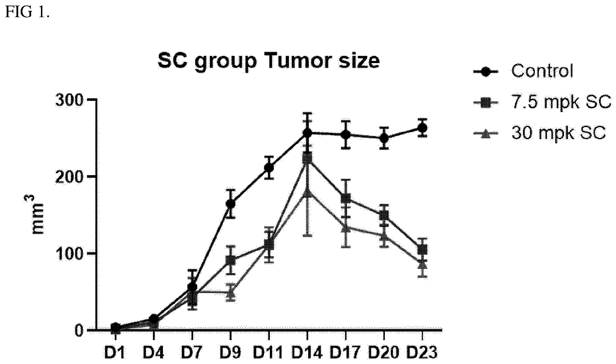

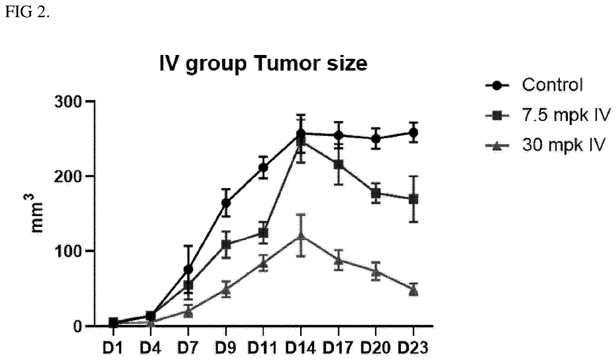

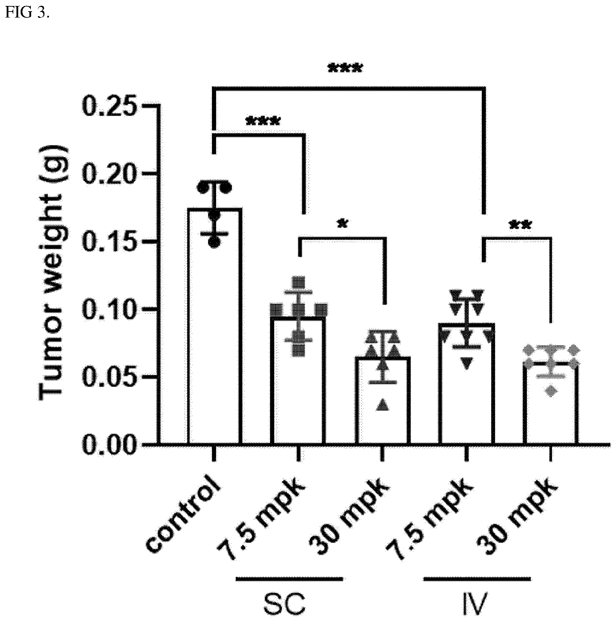

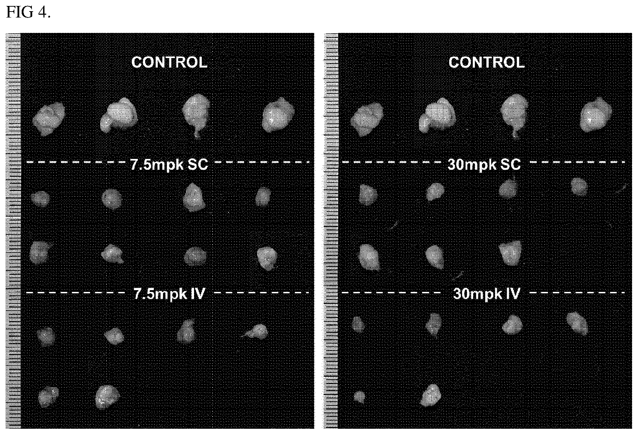

As a result of administering Compound 3 to the gastric cancer cell line SNU638 xenograft mouse model, it could be confirmed that Compound 3 exhibited a statistically significant cancer growth inhibitory effect in a concentration-dependent manner regardless of the route of administration thereof, and particularly, the effect became greater as it was administered for a longer period of time (FIGS. 1 to 4).

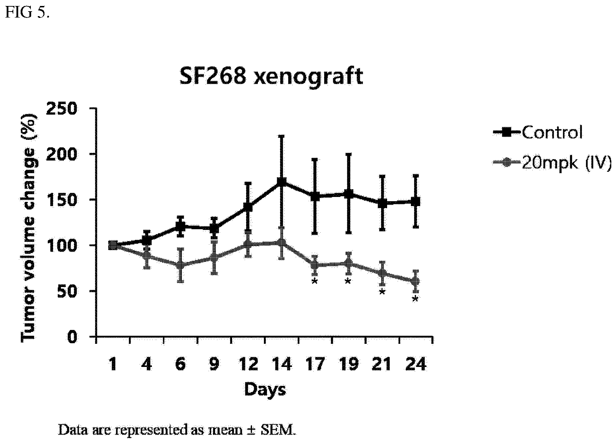

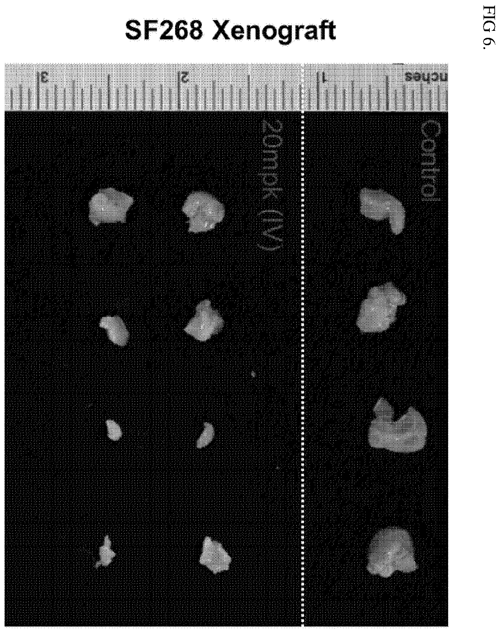

In addition, as a result of administering Compound 3 to the glioblastoma cell line SF638 xenograft mouse model by tail vein injection, it could be confirmed that Compound 3 exhibited a statistically significant cancer growth inhibitory effect at a concentration of 20 mpk, and particularly, the effect became greater as it was administered for a longer period of time (FIGS. 5 and 6).

So far, the present invention has been described with reference to the embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. An antisense compound comprising a modified oligonucleotide that is complementary to a nucleotide sequence in a transcript of a gene encoding WFDC2 (WAP Four-Disulfide Core Domain 2) and consists of 10 to 30 linked nucleosides.

2. The antisense compound of claim 1, wherein the nucleotide sequence in a transcript of a gene encoding WFDC2 is SEQ ID NO: 1 or SEQ ID NO: 2.

3. The antisense compound of claim 1, wherein the antisense compound comprises a modified oligonucleotide consisting of 16 to 20 linked nucleosides.

4. The antisense compound of claim 1, wherein the modified oligonucleotide comprises at least one modification selected from among at least one modified internucleoside linkage, at least one modified nucleoside comprising a modified sugar moiety, and at least one modified nucleoside comprising a modified nucleobase.

5. The antisense compound of claim 4, wherein the modified nucleoside is a modified nucleoside comprising at least one modified sugar moiety selected from the group consisting of sugar moieties substituted with 2'-O-methyl, 2'-O-methoxyethyl, 2'-amino, 2'-fluoro, 2'-arabino-fluoro, 2'-O-benzyl, or 2'-O-methyl-4-pyridine.

6. The antisense compound of claim 4, wherein the modified nucleoside is at least one modified nucleoside selected from the group consisting of locked nucleic acid (LNA), constrained ethyl bicyclic nucleic acid (cEt), 2'-O,4'-C-ethylene-bridged nucleic acid (ENA), and tricyclo-DNA.

7. The antisense compound of claim 4, wherein the modified nucleoside is a modified nucleoside comprising a sugar surrogate having a six-membered ring or an acyclic moiety.

8. The antisense compound of claim 4, wherein the modified nucleoside is a modified nucleoside comprising at least one modified nucleobase selected from the group consisting of pseudouridine, 2'-thiouridine, N6'-methyladenosine, 5'-methylcytidine, 5'-fluoro-2-deoxyuridine, N-ethylpiperidine 7'-EAA triazol modified adenine, N-ethylpiperidine 6'-triazol modified adenine, 6'-phenylpyrrolocytosine, 2',4'-difluorotoluylribonuleoside, and 5'-nitroindole.

9. The antisense compound of claim 4, wherein the modified internucleoside linkage is at least one modified internucleoside linkage selected from the group consisting of phosphotriester, phosphoramidate, mesyl phosphoramidate, phosphorothioate, phosphorodithioate, methylphosphonate, and methoxypropyl-phosphonate.

10. The antisense compound of claim 1, wherein the modified oligonucleotide comprises a gap segment consisting of linked deoxynucleosides, a 5' wing segment consisting of linked nucleosides, and a 3' wing segment consisting of linked nucleosides, wherein the gap segment is positioned between the 5' wing segment and the 3' wing segment and wherein the nucleoside of each wing segment comprises a modified sugar moiety or a sugar surrogate.

11. The antisense compound of claim 1, wherein the modified oligonucleotide comprise
a gap segment consisting of 8 to 10 linked deoxynucleosides;
a 5' wing segment consisting of 3 to 5 linked nucleosides; and
a 3' wing segment consisting of 3 to 5 linked nucleosides,
wherein the gap segment is positioned between the 5' wing segment and the 3' wing segment and wherein the nucleoside of each wing segment comprises a modified sugar moiety.

12. The antisense compound of claim 1, wherein the antisense compound comprises a modified oligonucleotide complementary to SEQ ID NO: 1 or SEQ ID NO: 2, wherein the antisense compound comprises the modified oligonucleotide with a nucleotide sequence comprising at least 8 contiguous nucleobases fully complementary to any portion of an oligonucleotide sequence selected from the group consisting of start site 25 to stop site 46, start site 284 to stop site 305, start site 520 to stop site 545, start site 2222 to stop site 2344, start site 7334 to stop site 9301, start site 9506 to stop site 9551, start site 9733 to stop site 10143, start site 10271 to stop site 10302, start site 10360 to stop site 10905, start site 10977 to stop site 11292, start site 11448 to stop site 11563, and start site 11633 to stop site 11773 of the nucleotide sequence of SEQ ID NO: 1, wherein the modified oligonucleotide reduces any one or more of mRNA level and protein level of WFDC.

13. The antisense compound of claim 1, wherein the antisense compound comprises a modified oligonucleotide complementary to SEQ ID NO: 1 or SEQ ID NO: 2, wherein the modified oligonucleotide has a nucleotide sequence comprising at least 8 contiguous nucleobases that perfectly match any one oligonucleotide sequence selected from the group consisting of SEQ ID NO: 7, SEQ ID NO: 8, SEQ ID NO: 9, SEQ ID NO: 20, SEQ ID NO: 38, SEQ ID NO: 39, SEQ ID NO: 42, SEQ ID NO: 43, SEQ ID NO: 46, SEQ ID NO: 47, SEQ ID NO: 48, SEQ ID NO: 49, SEQ ID NO: 65, SEQ ID NO: 83, SEQ ID NO: 86, SEQ ID NO: 89, SEQ ID NO: 109, SEQ ID NO: 113, SEQ ID NO: 119, SEQ ID NO: 120, SEQ ID NO: 121, SEQ ID NO: 122, SEQ ID NO: 123, SEQ ID NO: 129, SEQ ID NO: 131, SEQ ID NO: 135, SEQ ID NO: 136, SEQ ID NO: 140, SEQ ID NO: 141, SEQ ID NO: 142, SEQ ID NO: 148, SEQ ID NO: 154, SEQ ID NO: 155, SEQ ID NO: 156, SEQ ID NO: 157, SEQ ID NO: 165, SEQ ID NO: 169, SEQ ID NO: 176, SEQ ID NO: 191, SEQ ID NO: 205, SEQ ID NO: 210, SEQ ID NO: 213, SEQ ID NO: 214, SEQ ID NO: 215, SEQ ID NO: 218, SEQ ID NO: 228, SEQ ID NO: 229, SEQ ID NO: 237, SEQ ID NO: 238, SEQ ID NO: 239, SEQ ID NO: 240, SEQ ID NO: 243, SEQ ID NO: 244, SEQ ID NO: 245, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 264, SEQ ID NO: 282, SEQ ID NO: 284, SEQ ID NO: 285, SEQ ID NO: 286, SEQ ID NO: 287, SEQ ID NO: 289, SEQ ID NO: 290, SEQ ID NO: 291, SEQ ID NO: 291, SEQ ID NO: 292, SEQ ID NO: 293, SEQ ID NO: 295, SEQ ID NO: 300, SEQ ID NO: 310, SEQ ID NO: 313, SEQ ID NO: 314, SEQ ID NO: 316, SEQ ID NO: 317, SEQ ID NO: 320, SEQ ID NO: 330, SEQ ID NO: 331, SEQ ID NO: 336, SEQ ID NO: 343, SEQ ID NO: 376, SEQ ID NO: 377, SEQ ID NO: 378, SEQ ID NO: 379, SEQ ID NO: 380, SEQ ID NO: 381, SEQ ID NO: 382, and SEQ ID NO: 383, wherein the modified oligonucleotide reduces any one or more of mRNA level and protein level of WFDC.

14. The antisense compound of claim 1, wherein the antisense compound is a modified oligonucleotide having any one nucleotide sequence selected from the group consisting of SEQ ID NO: 7, SEQ ID NO: 8, SEQ ID NO: 9, SEQ ID NO: 20, SEQ ID NO: 38, SEQ ID NO: 39, SEQ ID NO: 42, SEQ ID NO: 43, SEQ ID NO: 46, SEQ ID NO: 47, SEQ ID NO: 48, SEQ ID NO: 49, SEQ ID NO: 65, SEQ ID NO: 83, SEQ ID NO: 86, SEQ ID NO: 89, SEQ ID NO: 109, SEQ ID NO: 113, SEQ ID NO: 119, SEQ ID NO: 120, SEQ ID NO: 121, SEQ ID NO: 122, SEQ ID NO: 123, SEQ ID NO: 129, SEQ ID NO: 131, SEQ ID NO: 135, SEQ ID NO: 136, SEQ ID NO: 140, SEQ ID NO: 141, SEQ ID NO: 142, SEQ ID NO: 148, SEQ ID NO: 154, SEQ ID NO: 155, SEQ ID NO: 156, SEQ ID NO: 157, SEQ ID NO: 165, SEQ ID NO: 169, SEQ ID NO: 176, SEQ ID NO: 191, SEQ ID NO: 205, SEQ ID NO: 210, SEQ ID NO: 213, SEQ ID NO: 214, SEQ ID NO: 215, SEQ ID NO: 218, SEQ ID NO: 228, SEQ ID NO: 229, SEQ ID NO: 237, SEQ ID NO: 238, SEQ ID NO: 239, SEQ ID NO: 240, SEQ ID NO: 243, SEQ ID NO: 244, SEQ ID NO: 245, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 264, SEQ ID NO: 282, SEQ ID NO: 284, SEQ ID NO: 285, SEQ ID NO: 286, SEQ ID NO: 287, SEQ ID NO: 289, SEQ ID NO: 290, SEQ ID NO: 291, SEQ ID NO: 291, SEQ ID NO: 292, SEQ ID NO: 293, SEQ ID NO: 295, SEQ ID NO: 300, SEQ ID NO: 310, SEQ ID NO: 313, SEQ ID NO: 314, SEQ ID NO: 316, SEQ ID NO: 317, SEQ ID NO: 320, SEQ ID NO: 330, SEQ ID NO: 331, SEQ ID NO: 336, SEQ ID NO: 343, SEQ ID NO: 376, SEQ ID NO: 377, SEQ ID NO: 378, SEQ ID NO: 379, SEQ ID NO: 380, SEQ ID NO: 381, SEQ ID NO: 382, and SEQ ID NO: 383.

15. A conjugate in which the antisense compound of any one of claims 1 to 14 is covalently linked to at least one non-nucleotide moiety.

16. The conjugate of claim 15, wherein the non-nucleotide moiety comprises a protein, a fatty acid chain, a sugar residue, a glycoprotein, a polymer, or any combinations thereof.

17. A pharmaceutical composition for preventing or treating cancer comprising the antisense compound of any one of claims 1 to 14 or the conjugate of any one of claims 15 and 16 as an active ingredient.

18. The composition of claim 17, wherein the cancer is selected from the group consisting of gastric cancer, esophageal cancer, bile duct cancer, ovarian cancer, cervical cancer, head and neck cancer, brain tumor, lung cancer, liver cancer, thyroid cancer, prostate cancer, bladder cancer, kidney cancer, gallbladder cancer, colorectal cancer, and pancreatic cancer.
